# EUROPEAN PATENT SPECIFICATION

(11) **EP 3 787 619 B1**
(45) Date of publication and mention of the grant of the patent: **11.05.2022**
(21) Application number: 19722552.7
(22) Date of filing: 30.04.2019
(51) Int. Cl.: A61K 31/4155, A61P 25/00, A61P 25/16

(54) **SERPIN INHIBITORS FOR THE TREATMENT OF PRION AND PRION-LIKE DISEASES**
SERPININHIBITOREN ZUR BEHANDLUNG VON PRIONEN UND PRIONENÄHNLICHEN ERKRANKUNGEN
INHIBITEURS DE LA SERPINE POUR LE TRAITEMENT DES MALADIES À PRIONS ET DE TYPE PRION

(30) Priority: 30.04.2018 EP 18170092
(43) Date of publication of application: 10.03.2021
(73) Proprietor: Scuola Internazionale Superiore di Studi Avanzati - SISSA, 34136 Trieste (IT); Fondazione Istituto Italiano di Tecnologia, 16163 Genova (IT); Forschungszentrum Jülich GmbH, 52425 Jülich (DE)
(72) Inventor: LEGNAME, Giuseppe, 34151 OPICINA (Trieste) (IT); VANNI, Silvia, 48018 Faenza (RA) (IT); CARLONI, Paolo, 52428 JÜLICH (DE); DE VIVO, Marco, 47922 RIMINI (IT)
(74) Representative: Rimini, Rebecca
(86) International application number: PCT/EP2019/061014
(87) International publication number: WO 2019/211265

(56) References cited:
- WO-A1-01/21591
- WO-A1-94/13643
- WO-A1-99/57101
- WO-A1-2008/074814
- S. VANNI ET AL: "Differential overexpression of SERPINA3 in human prion diseases", SCIENTIFIC REPORTS, vol. 7, no. 1, 15 November 2017 (2017-11-15), XP055514760, DOI: 10.1038/s41598-017-15778-8
- MAURA BARBISIN ET AL: "Gene expression profiling of brains from bovine spongiform encephalopathy (BSE)-infected cynomolgus macaques", BMC GENOMICS, BIOMED CENTRAL, vol. 15, no. 1, 5 June 2014 (2014-06-05), page 434, XP021187341, ISSN: 1471-2164, DOI: 10.1186/1471-2164-15-434

### SEQUENCE LISTING

<110> Scuola Internazionale Superiore di Studi Avanzati - SISSA; Fondazione
   Istituto Italiano di Tecnologia; Forschungszentrum Juelich GMBH
<120> SERPIN inhibitors for the treatment of prion and prion-like diseases
<130> PC1669RRI
<160> 3
<170> BiSSAP 1.3.6
<210> 1
   <211> 376
   <212> PRT
   <213> Homo sapiens
<220>
   <223> human plasminogen activator inhibitor PAI-1
<400> 1
   Ala Pro Ser Tyr Val Ala His Leu Ala Ser Asp Phe Gly Val Arg Val
   1               5                   10                  15
   Phe Gln Gln Val Ala Gln Ala Ser Lys Asp Arg Asn Val Val Phe Ser
               20                  25                  30
   Pro Tyr Gly Val Ala Ser Val Leu Ala Met Leu Gln Leu Thr Thr Gly
           35                  40                  45
   Gly Glu Thr Gln Gln Gln Ile Gln Ala Ala Met Gly Phe Lys Ile Asp
       50                  55                  60
   Asp Lys Gly Met Ala Pro Ala Leu Arg His Leu Tyr Lys Glu Leu Met
   65                  70                  75                  80
   Gly Pro Trp Asn Lys Asp Glu Ile Ser Thr Thr Asp Ala Ile Phe Val
                   85                  90                  95
   Gln Arg Asp Leu Lys Leu Val Gln Gly Phe Met Pro His Phe Phe Arg
               100                 105                 110
   Leu Phe Arg Ser Thr Val Lys Gln Val Asp Phe Ser Glu Val Glu Arg
           115                 120                 125
   Ala Arg Phe Ile Ile Asn Asp Trp Val Lys Thr His Thr Lys Gly Met
       130                 135                 140
   Ile Ser His Leu Leu Gly Thr Gly Ala Val Asp Gln Leu Thr Arg Leu
   145                 150                 155                 160
   Val Leu Val Asn Ala Leu Tyr Phe Asn Gly Gln Trp Lys Thr Pro Phe
                   165                 170                 175
   Pro Asp Ser Ser Thr His Arg Arg Leu Phe His Lys Ser Asp Gly Ser
               180                 185                 190
   Thr Val Ser Val Pro Met Met Ala Gln Thr Asn Lys Phe Asn Tyr Thr
           195                 200                 205
   Glu Phe Thr Thr Pro Asp Gly His Tyr Tyr Asp Ile Leu Glu Leu Pro
       210                 215                 220
   Tyr His Gly Asp Thr Leu Ser Met Phe Ile Ala Ala Pro Tyr Glu Lys
   225                 230                 235                 240
   Glu Val Pro Leu Ser Ala Leu Thr Asn Ile Leu Ser Ala Gln Leu Ile
                   245                 250                 255
   Ser His Trp Lys Gly Asn Met Thr Arg Leu Pro Arg Leu Leu Val Leu
               260                 265                 270
   Pro Lys Phe Ser Leu Glu Thr Glu Val Asp Leu Arg Lys Pro Leu Glu
           275                 280                 285
   Asn Leu Gly Met Thr Asp Met Phe Arg Gln Phe Gln Ala Asp Phe Thr
       290                 295                 300
   Ser Leu Ser Asp Gln Glu Pro Leu His Val Ala Leu Ala Leu Gln Lys
   305                 310                 315                 320
   Val Lys Ile Glu Val Asn Glu Ser Gly Thr Val Ala Ser Ser Ser Thr
                   325                 330                 335
   Ala Val Ile Val Ser Ala Arg Met Ala Pro Glu Glu Ile Ile Ile Asp
               340                 345                 350
   Arg Pro Phe Leu Phe Val Val Arg His Asn Pro Thr Gly Thr Val Leu
           355                 360                 365
   Phe Met Gly Gln Val Met Glu Pro
       370                 375
<210> 2
   <211> 394
   <212> PRT
   <213> Homo sapiens
<220>
   <223> human alpha-1-antitrypsin SERPINA1
<400> 2
   Glu Asp Pro Gln Gly Asp Ala Ala Gln Lys Thr Asp Thr Ser His His
   1               5                   10                  15
   Asp Gln Asp His Pro Thr Phe Asn Lys Ile Thr Pro Asn Leu Ala Glu
               20                  25                  30
   Phe Ala Phe Ser Leu Tyr Arg Gln Leu Ala His Gln Ser Asn Ser Thr
           35                  40                  45
   Asn Ile Phe Phe Ser Pro Val Ser Ile Ala Thr Ala Phe Ala Met Leu
       50                  55                  60
   Ser Leu Gly Thr Lys Ala Asp Thr His Asp Glu Ile Leu Glu Gly Leu
   65                  70                  75                  80
   Asn Phe Asn Leu Thr Glu Ile Pro Glu Ala Gln Ile His Glu Gly Phe
                   85                  90                  95
   Gln Glu Leu Leu Arg Thr Leu Asn Gln Pro Asp Ser Gln Leu Gln Leu
               100                 105                 110
   Thr Thr Gly Asn Gly Leu Phe Leu Ser Glu Gly Leu Lys Leu Val Asp
           115                 120                 125
   Lys Phe Leu Glu Asp Val Lys Lys Leu Tyr His Ser Glu Ala Phe Thr
       130                 135                 140
   Val Asn Phe Gly Asp Thr Glu Glu Ala Lys Lys Gln Ile Asn Asp Tyr
   145                 150                 155                 160
   Val Glu Lys Gly Thr Gln Gly Lys Ile Val Asp Leu Val Lys Glu Leu
                   165                 170                 175
   Asp Arg Asp Thr Val Phe Ala Leu Val Asn Tyr Ile Phe Phe Lys Gly
               180                 185                 190
   Lys Trp Glu Arg Pro Phe Glu Val Lys Asp Thr Glu Glu Glu Asp Phe
           195                 200                 205
   His Val Asp Gln Val Thr Thr Val Lys Val Pro Met Met Lys Arg Leu
       210                 215                 220
   Gly Met Phe Asn Ile Gln His Cys Lys Lys Leu Ser Ser Trp Val Leu
   225                 230                 235                 240
   Leu Met Lys Tyr Leu Gly Asn Ala Thr Ala Ile Phe Phe Leu Pro Asp
                   245                 250                 255
   Glu Gly Lys Leu Gln His Leu Glu Asn Glu Leu Thr His Asp Ile Ile
               260                 265                 270
   Thr Lys Phe Leu Glu Asn Glu Asp Arg Arg Ser Ala Ser Leu His Leu
           275                 280                 285
   Pro Lys Leu Ser Ile Thr Gly Thr Tyr Asp Leu Lys Ser Val Leu Gly
       290                 295                 300
   Gln Leu Gly Ile Thr Lys Val Phe Ser Asn Gly Ala Asp Leu Ser Gly
   305                 310                 315                 320
   Val Thr Glu Glu Ala Pro Leu Lys Leu Ser Lys Ala Val His Lys Ala
                   325                 330                 335
   Val Leu Thr Ile Asp Glu Lys Gly Thr Glu Ala Ala Gly Ala Met Phe
               340                 345                 350
   Leu Glu Ala Ile Pro Met Ser Ile Pro Pro Glu Val Lys Phe Asn Lys
           355                 360                 365
   Pro Phe Val Phe Leu Met Ile Glu Gln Asn Thr Lys Ser Pro Leu Phe
       370                 375                 380
   Met Gly Lys Val Val Asn Pro Thr Gln Lys
   385                 390
<210> 3
   <211> 401
   <212> PRT
   <213> Homo sapiens
<220>
   <223> human antichymotrypsin SERPINA3
<400> 3
   Met Ala Ser Asn Ser Pro Leu Asp Glu Glu Asn Leu Thr Gln Glu Asn
   1               5                   10                  15
   Gln Asp Arg Gly Thr His Val Asp Leu Gly Leu Ala Ser Ala Asn Val
               20                  25                  30
   Asp Phe Ala Phe Ser Leu Tyr Lys Gln Leu Val Leu Lys Ala Pro Asp
           35                  40                  45
   Lys Asn Val Ile Phe Ser Pro Pro Ser Ile Ser Thr Ala Leu Ala Phe
       50                  55                  60
   Leu Ser Leu Gly Ala His Asn Thr Thr Leu Thr Glu Ile Leu Lys Gly
   65                  70                  75                  80
   Leu Lys Phe Asn Leu Thr Glu Thr Ser Glu Ala Glu Ile His Gln Ser
                   85                  90                  95
   Phe Gln His Leu Leu Arg Thr Leu Asn Gln Ser Ser Asp Glu Leu Gln
               100                 105                 110
   Leu Ser Met Gly Asn Ala Met Phe Val Lys Glu Gln Leu Ser Leu Leu
           115                 120                 125
   Asp Arg Phe Thr Glu Asp Ala Lys Arg Leu Tyr Gly Ser Glu Ala Phe
       130                 135                 140
   Ala Thr Asp Phe Gln Asp Ser Ala Ala Ala Lys Lys Leu Ile Asn Asp
   145                 150                 155                 160
   Tyr Val Lys Asn Gly Thr Arg Gly Lys Ile Thr Asp Leu Ile Lys Asp
                   165                 170                 175
   Leu Asp Ser Gln Thr Met Met Val Leu Val Asn Tyr Ile Phe Phe Lys
               180                 185                 190
   Ala Lys Trp Glu Met Pro Phe Asp Pro Gln Asp Thr His Gln Ser Arg
           195                 200                 205
   Phe Tyr Leu Ser Lys Lys Lys Trp Val Met Val Pro Met Met Ser Leu
       210                 215                 220
   His His Leu Thr Ile Pro Tyr Phe Arg Asp Glu Glu Leu Ser Cys Thr
   225                 230                 235                 240
   Val Val Glu Leu Lys Tyr Thr Gly Asn Ala Ser Ala Leu Phe Ile Leu
                   245                 250                 255
   Pro Asp Gln Asp Lys Met Glu Glu Val Glu Ala Met Leu Leu Pro Glu
               260                 265                 270
   Thr Leu Lys Arg Trp Arg Asp Ser Leu Glu Phe Arg Glu Ile Gly Glu
           275                 280                 285
   Leu Tyr Leu Pro Lys Phe Ser Ile Ser Arg Asp Tyr Asn Leu Asn Asp
       290                 295                 300
   Ile Leu Leu Gln Leu Gly Ile Glu Glu Ala Phe Thr Ser Lys Ala Asp
   305                 310                 315                 320
   Leu Ser Gly Ile Thr Gly Ala Arg Asn Leu Ala Val Ser Gln Val Val
                   325                 330                 335
   His Lys Ala Val Leu Asp Val Phe Glu Glu Gly Thr Glu Ala Ser Ala
               340                 345                 350
   Ala Thr Ala Val Lys Ile Thr Leu Leu Ser Ala Leu Val Glu Thr Arg
           355                 360                 365
   Thr Ile Val Arg Phe Asn Arg Pro Phe Leu Met Ile Ile Val Pro Thr
       370                 375                 380
   Asp Thr Gln Asn Ile Phe Phe Met Ser Lys Val Thr Asn Pro Lys Gln
   385                 390                 395                 400
   Ala

## Description

### FIELD OF THE INVENTION

The present invention relates to compounds for use in the therapeutic treatment of a prion or prion-like disease and to pharmaceutical compositions comprising these compounds.

### BACKGROUND OF THE INVENTION

Over the last decades, the striking advancements in biomedical knowledge have led to a significant improvement of health conditions. This fact has ultimately led to the lengthening of life expectancy: by 2050, almost 400 million people will be ≥ 80 years old. As a consequence, the prevalence of neurodegenerative disorders is rapidly increasing. The fact of this augment is likely to represent a major social and economic issue in the near future, not only for high-income countries but also in low- and middle-income ones.

Even though great efforts have been dedicated to research for many years, the precise molecular mechanisms that lead to neurodegeneration in pathologies such as Alzheimer's, Parkinson's, Huntington's or Creutzfeldt-Jakob's diseases are far from being elucidated. In general, these disorders share a common mechanism of aberrant folding of distinct proteins that, upon misfolding, become more prone to aggregation, and, in turn, may become highly neurotoxic. This mechanism is referred to as "prion paradigm".

The physiological cellular prion protein (PrPC) is a membrane-bound protein predominantly expressed in the nervous tissue, where it probably plays a role in neuronal development and function. When in an aberrant misfolded state, the cellular prion proteins associate with one another, leading to protein aggregation, and impose their anomalous structure on benign PrP molecules. Prions thus act as corruptive templates (seeds) that initiate a chain-reaction of PrP misfolding and aggregation. As prions grow, fragment and spread, they cause neuronal loss, thereby perturbing the function of the nervous system and ultimately causing the death of the affected individual.

Prion diseases or transmissible spongiform encephalopathies (TSEs) are a class of fatal infectious neurodegenerative disorders whose pathogenesis mechanisms are not fully understood. Prion diseases are characterized by neuronal vacuolation and cell loss within the brain, which lead to the characteristic spongiform changes, namely holes in the brain, which give these diseases their name. These diseases manifest as sporadic, genetic or acquired. So far, neither specific biomarkers for early diagnosis nor effective therapeutic targets have been identified.

The PrP^{C} is encoded by the *PRNP* gene. Misfolding of this protein into the pathogenic PrP^{Sc} occurs in different prion diseases, and different types of human prion diseases are characterized by diverse PrP^{Sc} isoforms and by the polymorphisms present at PRNP codon 129. Mounting evidences suggest that in addition to gene coding for the PrP (PRNP) other genes may contribute to the genetic susceptibility of TSEs.

The misfolding and aggregation of specific proteins within nervous system occur also in neurodegenerative diseases such as Alzheimer's disease (AD), Parkinson's disease (PD), Huntington's disease (HD) and frontotemporal dementia (FTD), now referred as prion-like disorders. In these disorders, Tau, beta-amyloid, and alpha-synuclein are the most commonly misfolded proteins, which form highly ordered filamentous inclusions with a core region of cross-betaconformation. These proteins have also been named 'prionoids' indicating that they exhibit prion-like properties.

Prion diseases affect also a wide range of animal species, most notably ruminants. Scrapie, a transmissible spongiform encephalopathy (TSE) in sheep, was first described in the eighteenth century and bovine spongiform encephalopathy (BSE), better known as mad cow disease, is among the many recently discovered zoonotic diseases. Most prion diseases in animals have basically occurred via the acquisition of infection from contaminated feed or via the exposure to contaminated environment. The realization that BSE is likely responsible for a new form of TSE in humans (variant Creutzfeldt-Jakob disease or vCJD), thereby indicating that prion diseases can cross species barriers from animals to humans, had posed a significant threat to human health. Therefore, the control of these disorders necessitates the development of efficacious therapeutics in order to achieve blocking of the cellular spread of infectivity or the propagation of prions.

Recent advances in the diagnosis of prion and prion-like diseases primarily rely on the identification and validation of disease-specific biomarkers. The scientific article Barbisin, M. et al. "Gene expression profiling of brains from bovine spongiform encephalopathy (BSE)-infected cynomolgus macaques" BMC Genomics 2014 15, 434, describes a first large-scale transcriptome gene expression analysis on BSE-infected cynomolgus macaques (Macaca fascicularis), which revealed a striking upregulation of the SERPINA3 protein in the frontal cortex of intracranially infected monkeys.

The researches described in Vanni S. et al "Differential overexpression of SERPINA3 in human prion diseases" Sci Rep. 2017 Nov 15;7(1):15637, on brain tissue samples of patients with different prion diseases revealed a strong increase in the expression of the serine protease inhibitor SERPINA3, both at the mRNA and protein level, in comparison with control subjects. This study further confirmed in various human prion disorders the SERPINA3 dysregulation pattern which had been previously highlighted in macaques. The authors of this publication suggest the use of SERPINA3 as a potential biomarker for human prion diseases, particularly as a tool for distinguishing different forms of these disorders.

To date, despite numerous active efforts, there are no drugs available for the cure of the neurodegenerative diseases collectively referred as prion and prion-like disorders. Symptomatic treatment is the only available option, including the administration to patients of antipsychotics, such as quetiapine and clonazepam, to treat myoclonus, and of selective serotonin re-uptake inhibitors (SSRIs) to treat depression. Currently, drug discovery efforts aiming at the identification of compounds for the therapeutic treatment of prion diseases have been following the so-called 'small molecule design' approach, whose rationale relies in the ability of a given compound to interfere with PrP^{C} conversion into PrP^{Sc}, thereby blocking selfamplification of misfolded proteins and, consequently, halting the pathology spreading. However, despite the generation of a large number of compound libraries, all tested molecules have failed to prove effective when administered to humans.

It is therefore an object of the present invention to provide a medicament having activity against prion or prion-like diseases in a human or animal subject.

It is another object of the present invention to provide a medicament which is effective from an early stage of a prion or prion-like disease.

Further, an object of the present invention is to provide a medicament which is effective in preventing or delaying the onset of a prion or prion-like disease.

A yet further object of the present invention is to provide a medicament which is effective in inhibiting the progression of already established prion or prion-like disease-associated neurodegenerative processes.

### SUMMARY OF THE INVENTION

The present invention provides a SERPINA3 inhibitor compound for use in the prevention and/or therapeutic treatment of a prion or prion-like disease in a subject in need thereof.

The SERPINA3 inhibitor compound for use according to the invention has the general formula (I) 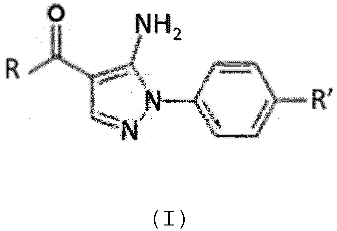 wherein
R is a saturated or unsaturated 5- or 6-membered ring containing at least one heteroatom; and
R' is a halogen atom or a C₁-C₄ alkyloxy group substituted with at least one halogen atom.

Preferably, the heteroatom is nitrogen.

A halogen atom is selected from the group consisting of fluorine (F), iodine (I), chlorine (Cl) and bromine (Br). More preferably, the halogen atom is fluorine.

A C₁-C₄ alkyloxy group is selected from the group consisting of methoxy, ethoxy, propyloxy, iso-propyloxy, n-butyloxy, iso-butyloxy, sec-butyloxy and terzbutyloxy.

In a particularly preferred embodiment, R in general formula (I) is a piperidine or pyrrole ring and/or R' is a fluorine atom or a trifluoromethoxy group.

In a more preferred embodiment, the SERPINA3 inhibitor compound for use according to the invention has the formula (II):

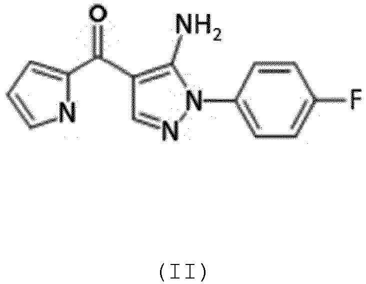

In another more preferred embodiment, the SERPINA3 inhibitor compound for use according to the invention has the formula (III)

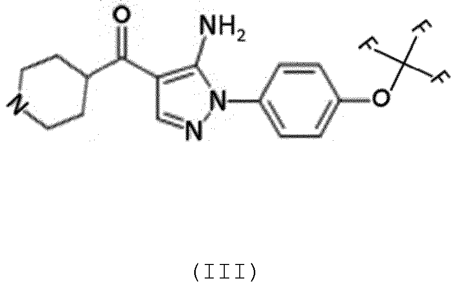

In one embodiment, the invention provides the use of the SERPINA3 inhibitor compound of general formula (I) as above defined for affecting the onset and/or progression of the prion or prion-like disease.

The invention also provides a pharmaceutical composition for use in the prevention and/or therapeutic treatment of a prion or prion-like disease in a subject in need thereof. The pharmaceutical composition contains the SERPINA3 inhibitor compound of general formula (I) as above defined in combination with a pharmaceutically acceptable vehicle, excipient and/or diluent.

Other features and advantages of the SERPINA3 inhibitor compound for use according to the invention are defined in the appended claims which form an integral part of the description.

### DESCRIPTION OF THE DRAWINGS

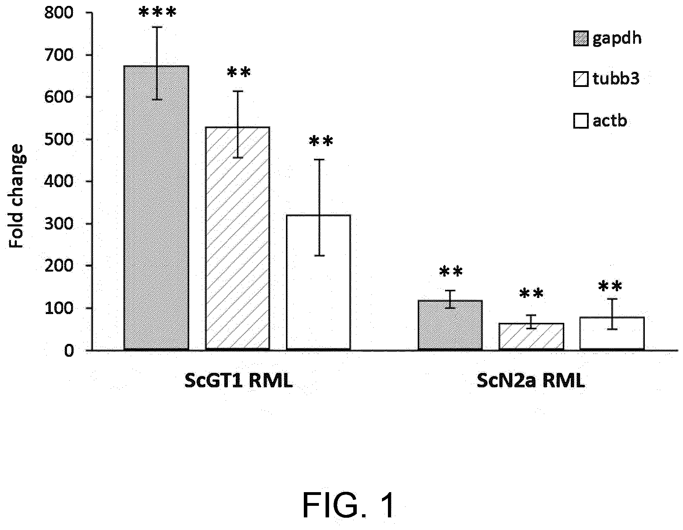

Figure 1 is a graph showing relative fold changes of Serpina3n mRNA in GT1 and N2a cell lines infected with prion strain RML (ScGT1 and ScN2a) compared to non-infected GT1 and N2a cells, respectively (n=5 for each group). The fold change was normalized with *gapdh, tubb3 and actb.* *** p<0.00000005; ** p<0.00001; *p<0.005

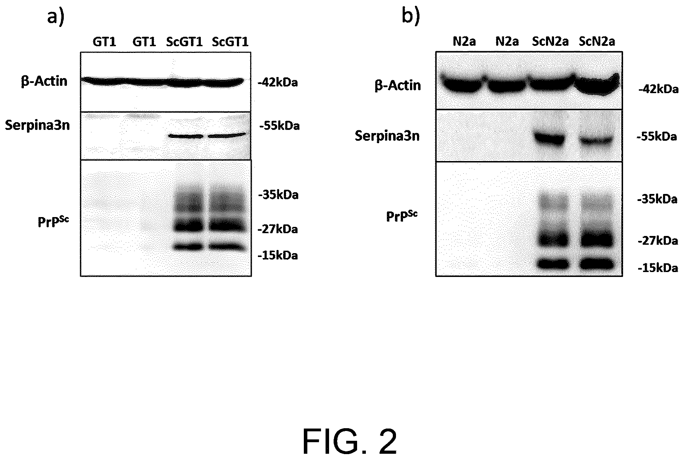

Figure 2 shows the results of Western blotting analysis of Serpina3n and PrP^{Sc} in lysates from chronically RML-infected a) GT1 (ScGT1) and b) N2a (ScN2a) cells. β-actin was used as proteins loading control. Molecular weight is represented on the right (kDa).

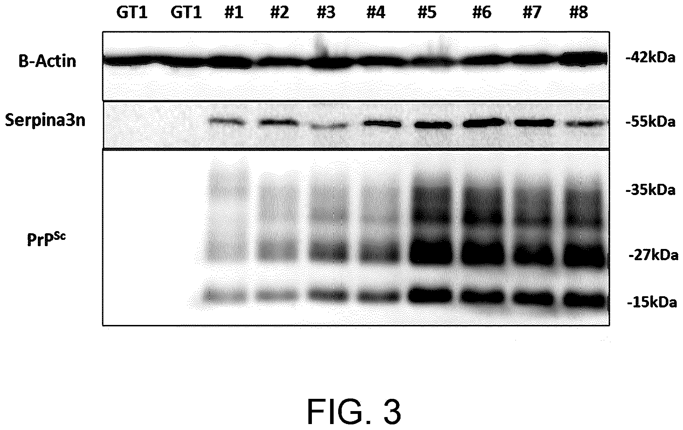

Figure 3 shows the results of Western blotting analysis of lysates from parental GT1 cells and de novo RML-infected cells (ScGT1, passages #1 - #8). β-actin was used as proteins loading control. Molecular weight is represented on the right (kDa).

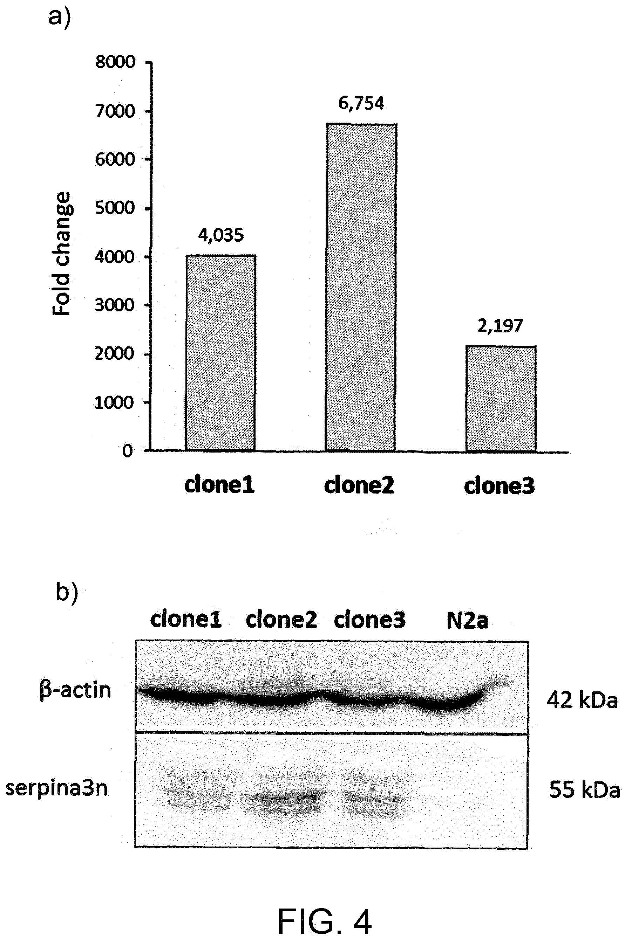

Figure 4 shows the results of real-time PCR (a) and Western blotting (b) analysis on N2a clones 1 - 3 which stably overexpress Serpina3n. a) Graph illustrating relative fold changes of Serpina3n mRNA in prion-infected N2a clones 1-3, compared to parental N2a cells. b) Image showing the results of Western blotting analysis of Serpina3n in lysates from N2a clones 1-3 and parental N2a cells. β-actin was used as proteins loading control. Molecular weight is represented on the right (kDa).

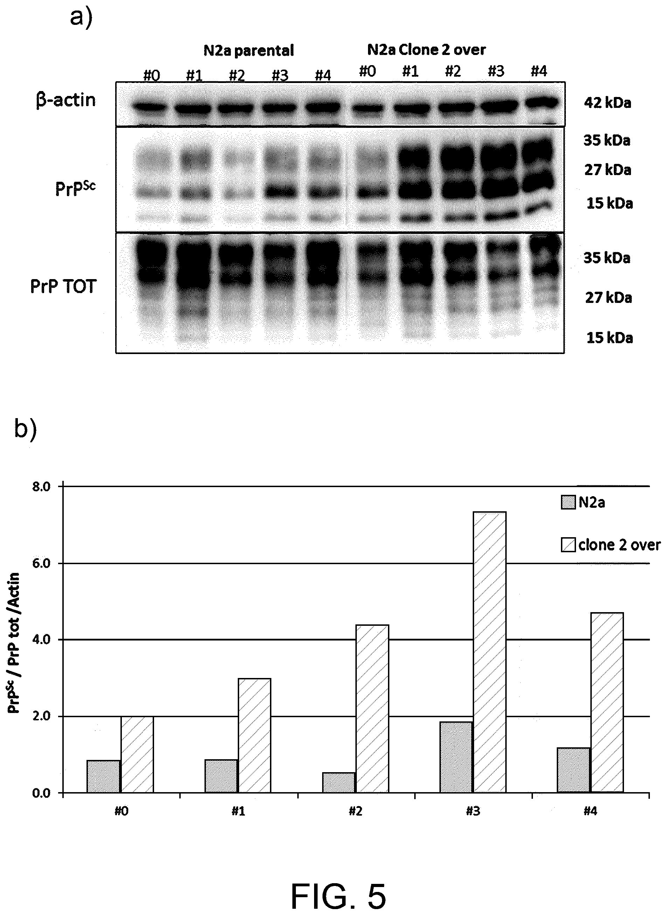

Figure 5 shows PrP^{Sc} accumulation in de novo prion-infected cells overexpressing Serpina3n. a) Western blotting analysis of lysates from de novo prion-infected parental N2a cells (passages #1 - #4) and N2a clone 2 stably overexpressing Serpina3n (passages #1 - #4). Molecular weight is represented on the right (kDa). b) Graph illustrating the results of densitometric analysis. β-actin was used as proteins loading control and to normalize the expression level of PrP^{Sc} for densitometric analysis.

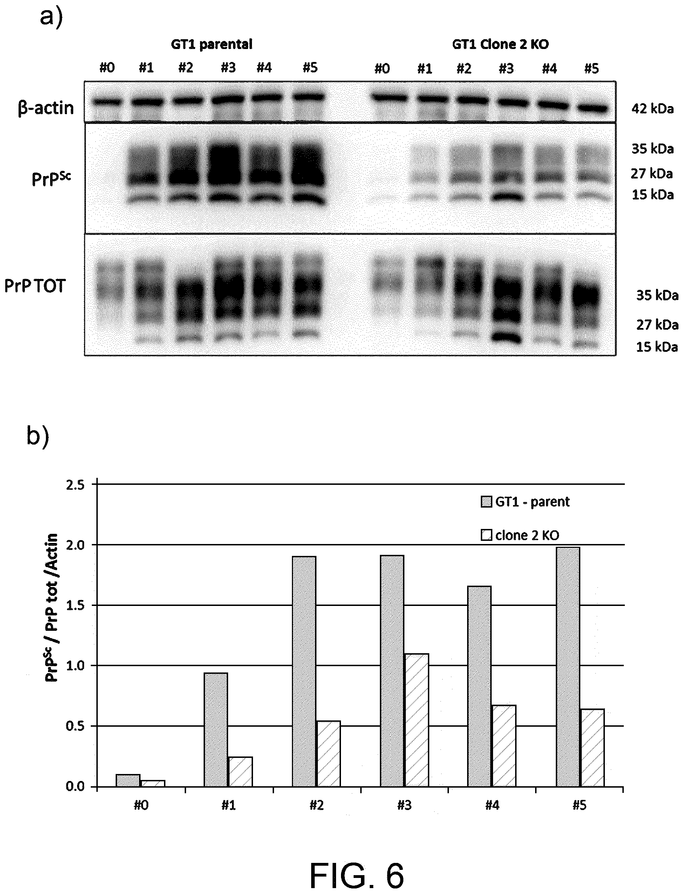

Figure 6 shows PrP^{Sc} decrease in Serpina3n knock-out cells upon de novo infection with prions. a) Western blotting analysis of lysates from de novo prion-infected parental GT1 cells (passages #1 - #5) and GT1 clone 2 wherein Serpina3n expression has been stably knock-out (passages #1 - #5). Molecular weight is represented on the right (kDa). b) Graph illustrating the results of densitometric analysis. β-actin was used as proteins loading control and to normalize the expression level of PrP^{Sc} for densitometric analysis.

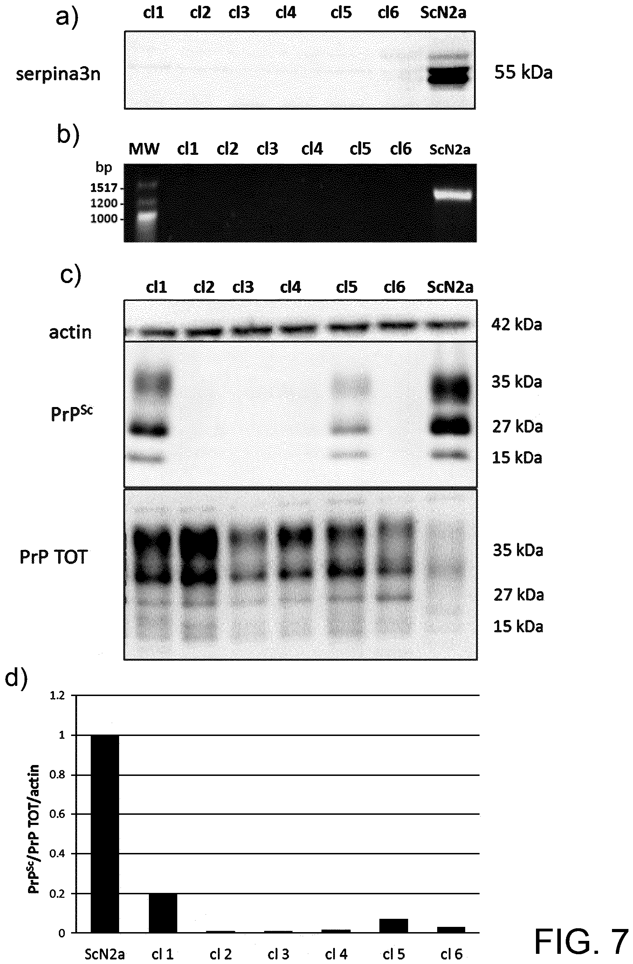

Figure 7 shows the results of experiments carried out on Serpina3n knock-out N2a cells chronically infected with prions (ScN2a). a) Western blotting analysis of Serpina3n in lysates from parental ScN2a cells and Serpina3n knock-out N2a clones 1-6. Molecular weight is represented on the right (kDa). b) PCR analysis of Serpina3n expression in mRNA samples from Serpina3n knock-out N2a clones. Serpina3n coding sequence = 1256 bp. c) Western blotting analysis of PrP^{Sc} in lysates from parental ScN2a cells and Serpina3n knock-out N2a clones 1-6. Molecular weight is represented on the right (kDa). d) Graph illustrating the results of PrP^{Sc} densitometric analysis. β-actin was used as proteins loading control and to normalize the expression level of PrP^{Sc} for densitometric analysis.

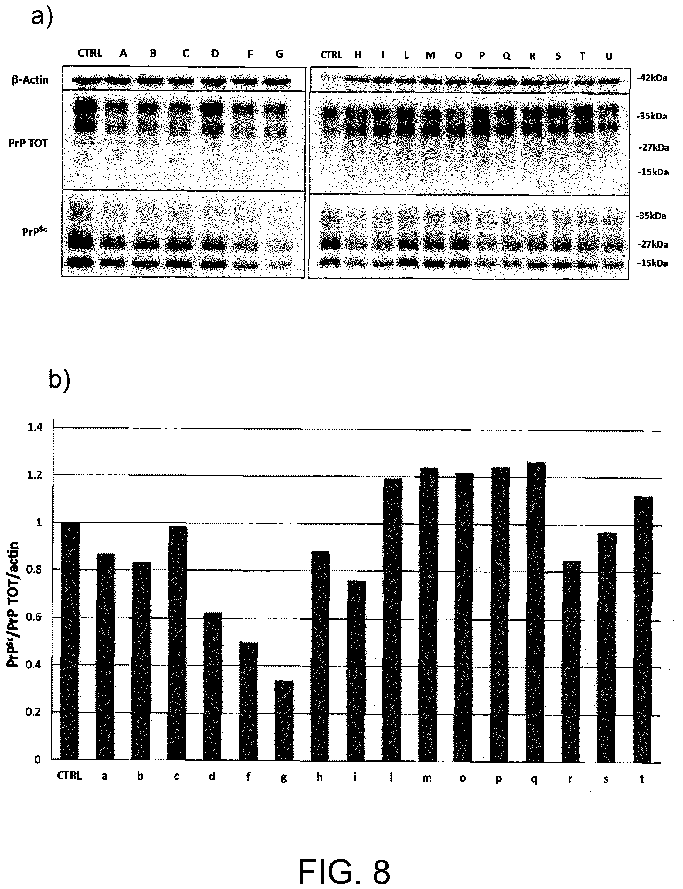

Figure 8 shows the results on PrP^{Sc} levels of treating GT1 cells chronically infected with RML prion (ScGT1) with SERPINA3 inhibitor compounds from the first library. a) Western blotting analysis of PrP^{Sc} in lysates from ScGT1 treated with SERPINA3 inhibitor compounds from the first library (compounds A-U) or with the vehicle as control (CTRL). Molecular weight is represented on the right (kDa). b) Graph illustrating the results of PrP^{Sc} densitometric analysis. β-actin was used as proteins loading control and to normalize the expression level of PrP^{Sc} for densitometric analysis.

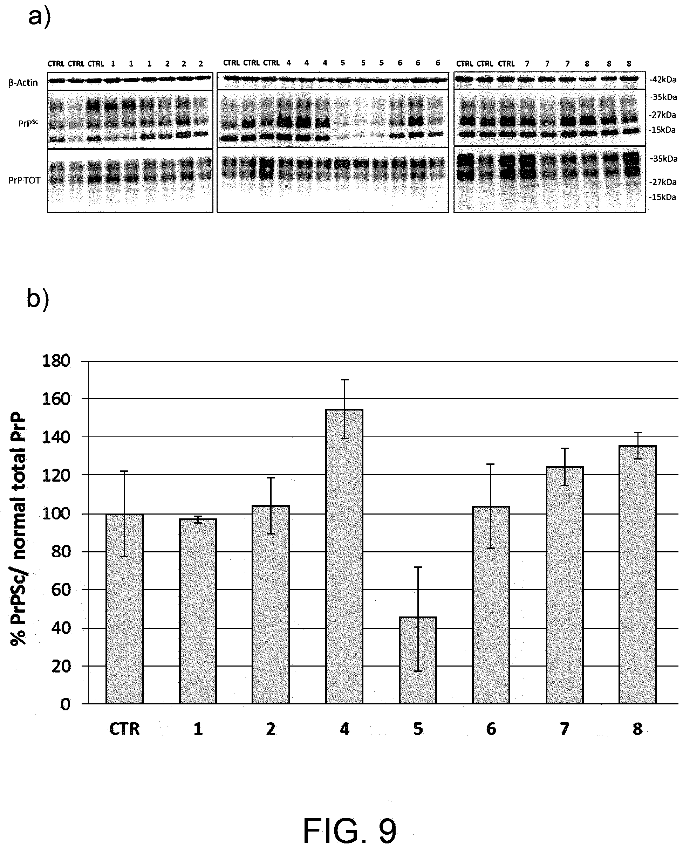

Figure 9 shows the results on PrP^{Sc} levels of treating GT1 cells chronically infected with RML prion (ScGT1) with SERPINA3 inhibitor compounds from the second library. a) Western blotting analysis of PrP^{Sc} in lysates from ScGT1 treated with SERPINA3 inhibitor compounds from the second library (compounds 1-8) or with the vehicle as control (CTRL). The experiments have been performed in triplicates. Molecular weight is represented on the right (kDa). b) Graph illustrating the results of PrP^{Sc} densitometric analysis. β-actin was used as proteins loading control and to normalize the expression level of PrP^{Sc} for densitometric analysis.

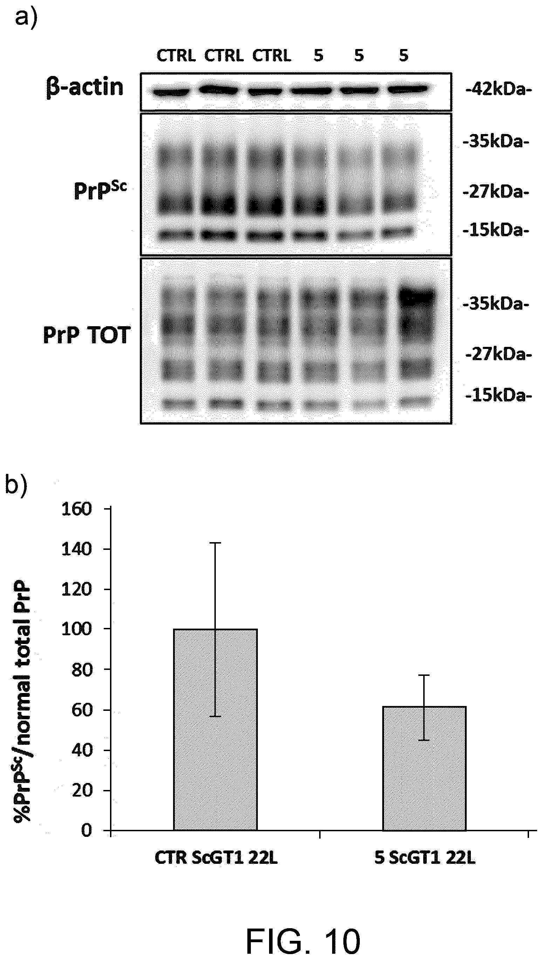

Figure 10 shows that compound 5 of formula (III) is effective on GT1 cells chronically infected with 22L prion strain (ScGT1 22L). a) Western blotting analysis of PrP^{Sc} in lysates from ScGT1 22L cells treated with compound 5 of formula (III) from the second library or with the vehicle as control (CTRL) . The experiment has been performed in triplicates. Molecular weight is represented on the right (kDa). b) Graph illustrating the results of PrP^{Sc} densitometric analysis. β-actin was used as proteins loading control and to normalize the expression level of PrP^{Sc} for densitometric analysis.

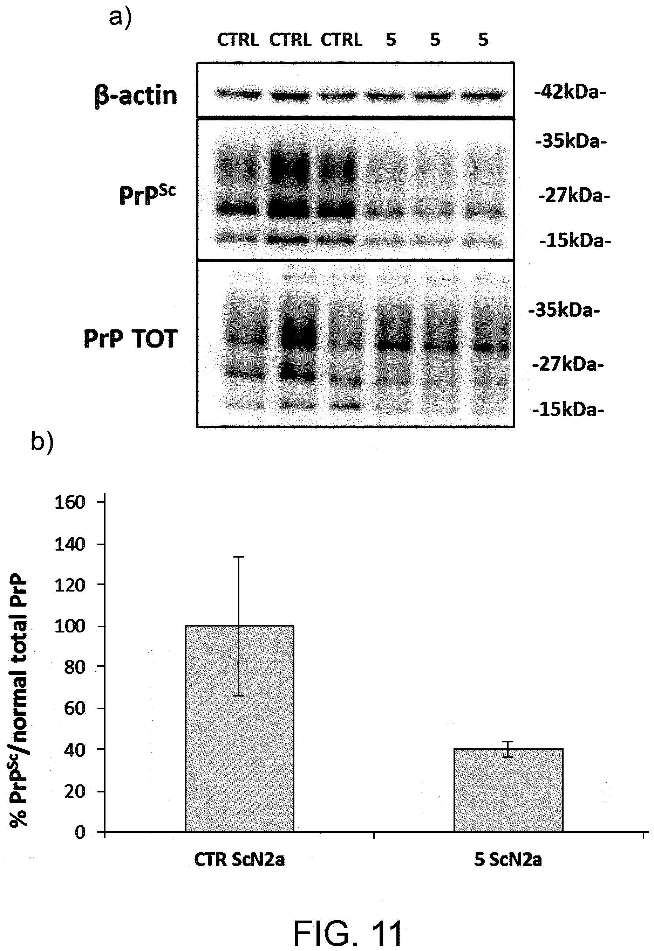

Figure 11 shows that compound 5 of formula (III) is effective on N2a cells chronically infected with RML prion strain (ScN2a). a) Western blotting analysis of PrP^{Sc} in lysates from ScN2a cells treated with compound 5 of formula (III) from the second library or with the vehicle as control (CTRL). The experiment has been performed in triplicates. Molecular weight is represented on the right (kDa). b) Graph illustrating the results of PrP^{Sc} densitometric analysis. β-actin was used as proteins.

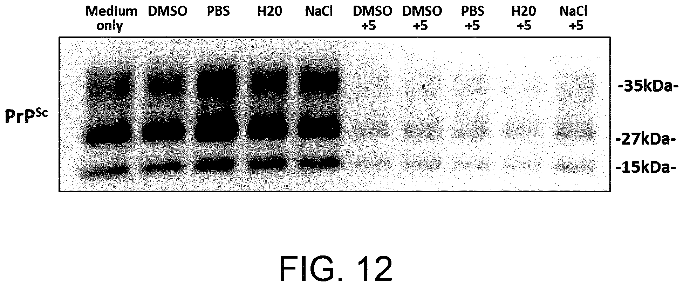

Figure 12 shows the results of Western blotting analysis of PrP^{Sc} in lysates from untreated ScN2a cells (medium only), ScN2a cells treated with the vehicle only (DMSO, PBS, H₂0 and NaCl) or with compound 5 of formula (III) dissolved in each of the vehicles. Molecular weight is represented on the right (kDa).

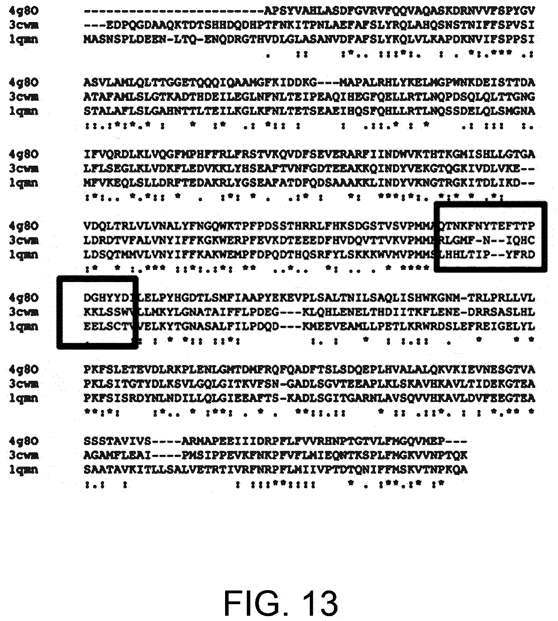

Figure 13 shows a multiple alignment of the amino acid sequences of the human plasminogen activator inhibitor PAI-1 complexed with CDE-096 (PDB ID: 4G8O), the alpha-1-antitrypsin SERPINA1 complexed with citrate (PDB ID: 3CWM) and the human antichymotrypsin SERPINA3 (1QMN). The alignment reveals that the sB/sC pocket (black squares), which is the ligand interacting site, is one of the least conserved regions among the examined proteins.

### DETAILED DESCRIPTION OF THE INVENTION

It should be noted that, as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", "characterized by" and "having" can be used interchangeably.

Unless defined otherwise, all technical and scientific terms used herein have the same meanings as commonly understood by one of ordinary skill in the art to which this invention belongs.

As used herein, the term "subject" is meant to refer to an animal. The subject can be a vertebrate, more preferably a mammal. Mammals include, but are not limited to, primates, bovids, ovids, ungulates, felids, cervids. A mammal can be, for example, a human being, a monkey, a cow, a sheep, a goat, a dog, a cat, a mink, a deer, an elk a pig, a mouse, a rat. In accordance to the present invention, the subject can be affected by a prion or prion-like disease, exhibiting symptoms which may vary from wild to severe. Alternatively, according to the present invention, the subject can be at risk of developing a prion or prion-like disease.

As used herein, unless otherwise specified, the term "treating" refers to the administration of the compound of the invention after the onset of symptoms of the prion or prion-like disease whereas "preventing" refers to the administration prior to the onset of the symptoms, particularly to subjects at risk of the disease.

As used herein, the term "affect" refers to either blocking or slow downing the pathological processes associated with prion or prion-like disorders.

As used herein, the term "therapeutically effective amount" refers to an amount of the compound for use according to the invention sufficient to exhibit a detectable therapeutic effect. The precise effective amount for a subject will depend upon the subject's size and health, the nature and severity of the condition to be treated.

The present inventors have found that, upon prion infection, the accumulation of misfolded prion protein PrP^{Sc} is significantly decreased in neural cells wherein the expression of SERPINA3 protein had been stably knock-out (Figures 6 and 7). These findings are in agreement with the results of experiments carried out by the inventors on neural cells modified to stably overexpress SERPINA3, wherein the amount of cellular PrP^{Sc} induced by prion infection highly correlates with the increase in SERPINA3 expression levels across time (Figure 5).

SERPINA3 is an alpha globulin glycoprotein, which is a member of the serine protease inhibitor family, able to inhibit several proteases, including mast cell chymase, pancreatic chymotrypsin, human glandular kallikrein 2, kallikrein 3 (prostate-specific antigen), and pancreatic cationic elastase. It is a typical acute-phase protein secreted into the circulation during acute and chronic inflammation. High levels of SERPINA3 have also been reported in several malignant melanomas and carcinomas where it might have a role in regulating apoptosis and invasiveness.

Based on these findings, the inventors have carried out experimental studies in order to exploit the role of the SERPINA3 protein as a potential target for drug design in therapeutic approaches aiming at contrasting the neurodegenerative processes which are the common hallmark of prion and prion-like disorders. As a first step, chemical compounds potentially acting as inhibitor of SERPINA3 activity have been selected by performing an *in silico* screening on several compound libraries for their ability to interact with the protein structure. Briefly, a drug target site has been identified on SERPINA3 by superimposing the protein structure with the known structures of other two molecules belonging to the Serpin family, which have been crystallized with their respective inhibitors. Following this screening, the effects of the compounds thus selected have been tested in in vitro cellular models of prion diseases. As shown in Figure 8, of the SERPINA3 inhibitors administered to prion-infected neural cells, only one of them achieved a dramatic decrease in prion load by drastically reducing the levels of the misfolded form of the prion protein, compared to the vehicle alone. Because of the strong therapeutic potential shown by this SERPINA3 inhibitor, this compound, which in the following is designated as compound G, was selected for further analysis.

To further improve drug efficacy, the chemical structure of compound G was used as a model to generate a library of structural analog compounds. The activity of these structural analogs have been assayed in vitro on neural cells infected with different prion strains, revealing that only one of them, hereinafter designated as compound number 5, was effective against the formation and accumulation of PrP^{Sc} across the range of prion strains tested, thereby leading to a significant reduction in prion load (Figures 9 - 11).

Surprisingly, the present inventors have found that the SERPINA3 inhibitor compounds whose therapeutic efficacy have been proven in vitro against misfolded proteins formation and accumulation, are illustrated by the general formula (I): 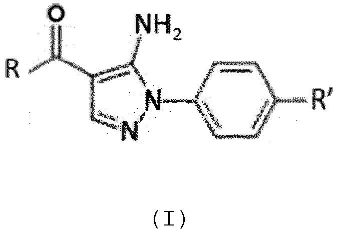 wherein
R is a saturated or unsaturated 5- or 6-membered ring containing at least one heteroatom; and
R' is a halogen atom or a C₁-C₄ alkyloxy group substituted with at least one halogen atom.

Therefore, an aspect of the present invention is a SERPINA3 inhibitor compound, for use in the therapeutic treatment of a prion or prion-like disease in a subject in need thereof, said compound having the above defined general formula (I).

Preferably, in the general formula (I) the heteroatom is nitrogen.

A halogen atom is selected from the group consisting of fluorine (F), iodine (I), chlorine (Cl) and bromine (Br). More preferably, the halogen atom is fluorine.

A C₁-C₄ alkyloxy group is selected from the group consisting of methoxy, ethoxy, propyloxy, iso-propyloxy, n-butyloxy, iso-butyloxy, sec-butyloxy and terzbutyloxy.

In a particularly preferred embodiment, R in general formula (I) is a piperidine or pyrrole ring and/or R' is a fluorine atom or a trifluoromethoxy group.

In a more preferred embodiment, the SERPINA3 inhibitor compound for use according to the invention has the formula (II):

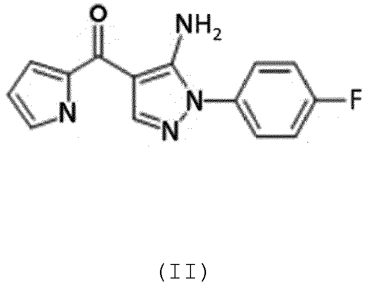

In another more preferred embodiment, the SERPINA3 inhibitor compound for use according to the invention has the formula (III)

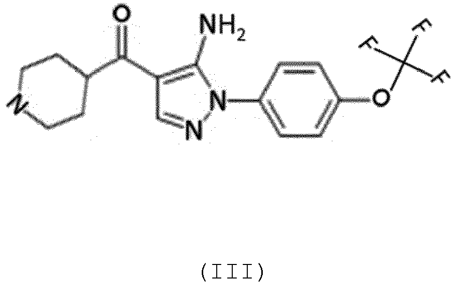

According to the present invention, compound G is illustrated by the structural formula (II) and compound 5 is illustrated by the structural formula (III).

Accordingly, in the present description, the compound of formula (II) is also designated as compound G, and the compound of formula (III) is also designated as compound 5.

Advantageously, the therapeutic use of the SERPINA3 inhibitor compound against prion diseases enables to achieve a significant decrease in the conversion and/or accumulation of the misfolded prion protein without interfering with the activity of the cellular PrP^{C} isoform. This protein is known to play an important physiological role in the central nervous system.

Moreover, since the compound for use according to the invention targets a protease inhibitor, which may be responsible for defective misfolded protein clearance, and acts independently from the particular structure of the misfolded protein, a therapeutic strategy based on the use of this compound is effective also in the treatment of prion-like neurodegenerative diseases which share the same protein misfolding mechanism.

Without wishing to be bound by any theory, the inventors believe that the SERPINA3 protein present in the brain tissues of diseased subjects may lead to the inhibition of the target protease(s) that, in physiological conditions, would degrade aberrant misfolded proteins such as PrP^{Sc} in the prion disease. By reducing the activity of the SERPINA3 protease inhibitor, the physiological intracellular function of the target protease(s) is restored, which, being active again, might effectively degrade misfolded proteins, thereby slowing down the progression of prion or prion-like diseases.

As further illustrated in the Experimental Section below, the present inventors have found that the appearance and accumulation of misfolded protein strongly correlate with the rapid increase in the expression levels of the protein SERPINA3 in neural cells from very early passages after prion infection. These findings highlight the role of SERPINA3 as a therapeutic target for early intervention in prion and prion-like disorders and indicate that the administration of the compound of the invention at an early disease stage is effective to achieve a delay in the disease onset, or to prevent the disease itself.

A preferred embodiment of the present invention relates to a therapeutic method aiming at affecting the onset and/or progression of the prion or prion-like disease in a subject.

Human prion or prion-like diseases, associated with abnormal protein misfolding, that can be treated with the SERPINA3 inhibitor compound for use according to the invention include, but are not limited to, Creutzfeldt-Jacob disease (CJD), fatal familial insomnia (FFI), Gerstmann-Straussler-Scheinker syndrome (GSS), Alzheimer's disease, Parkinson's disease, Huntington's Chorea, Amyotrophic lateral sclerosis (ALS) and Frontotemporal Dementia (FTD).

Exemplary animal prion or prion-like diseases include, but are not limited to, feline spongiform encephalopathy of zoological and domestic cats (FSE) and transmissible spongiform encephalopathy (TSE) of zoological ruminants and non-human primates, as well as chronic wasting disease of deer and elk (CWD) and transmissible mink encephalopathy of farmed mink (TME).

The exact dose of the SERPINA3 inhibitor compound for use according to the invention may vary depending on the targeted disease as well as on the subject's characteristics (e.g. sex, age, weight, etc.). The effective amount for a given situation can be determined by routine experimentation.

The SERPINA3 inhibitor compound for use according to the invention may also be effectively administered in the form of a pharmaceutical composition.

Accordingly, a second aspect of the present invention is a pharmaceutical composition for use in the therapeutic treatment of a prion or prion-like disease, comprising a therapeutically effective amount of a SERPINA3 inhibitor compound and pharmaceutically acceptable vehicles, excipients and/or diluents, wherein said SERPINA3 inhibitor compound has the formula (I): 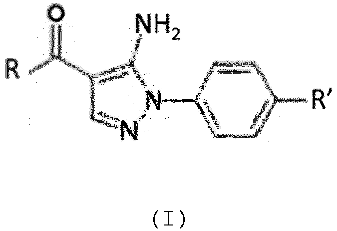 wherein
R is a saturated or unsaturated 5- or 6-membered ring containing at least one heteroatom; and
R' is a halogen atom or a C₁-C₄ alkyloxy group substituted with at least one halogen atom.

Preferably, the heteroatom is nitrogen.

A halogen atom is selected from the group consisting of fluorine (F), iodine (I), chlorine (Cl) and bromine (Br). More preferably, the halogen atom is fluorine.

A C₁-C₄ alkyloxy group is selected from the group consisting of methoxy, ethoxy, propyloxy, iso-propyloxy, n-butyloxy, iso-butyloxy, sec-butyloxy and terzbutyloxy.

In a particularly preferred embodiment, R in general formula (I) is a piperidine or pyrrole ring and/or R' is a fluorine atom or a trifluoromethoxy group.

In a more preferred embodiment, the SERPINA3 inhibitor compound in the pharmaceutical composition for use according to the invention has the formula (II):

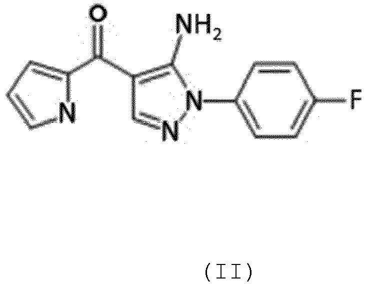

In another more preferred embodiment, the SERPINA3 inhibitor compound for use according to the invention has the formula (III)

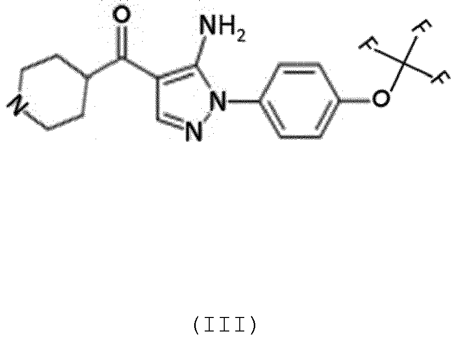

In a pharmaceutical composition, the amount of the administered active ingredient can vary widely according to considerations such as the particular compound and dosage unit employed, the mode and time of administration, the period of treatment, the age, sex, and general condition of the subject treated, the nature and extent of the condition treated, the rate of drug metabolism and excretion, the potential drug combinations and drug-drug interactions.

The pharmaceutical composition for use according to the invention is suitable to be administered as therapy against a prion or prion-like disease to any vertebrate, including human beings. Modifications of pharmaceutical compositions suitable for administration to humans in order to render the compositions suitable for administration to various animals are well known, and the person of ordinary skill in the art can design and perform such modifications by using his/her normal knowledge.

The term "pharmaceutically acceptable" refers to compounds and compositions which may be administered to mammals without undue toxicity at concentrations consistent with effective activity of the active ingredient. Pharmaceutically acceptable carriers (excipients) can for example be fillers, disintegrants, glidants and lubricants. Disintegrants are compounds which enhance the ability of the dosage form, such as tablet, to break into smaller fragments when in contact with a liquid, preferably water. Glidants can be used to improve the drug flowability. Suitable glidants are for example colloidal silicon dioxide, talcum or mixtures thereof. Lubricants generally can be regarded as substances which are suitable to reduce friction, such as static friction, sliding friction and rolling friction. Exemplary pharmaceutically acceptable salts include mineral acid salts such as hydrochlorides, hydrobromides, phosphates and sulfates; and the salts of organic acids such as acetates, propionates, malonates or benzoates.

Preferably, the SERPINA3 inhibitor compound for use according to the invention is dissolved in a liquid vehicle selected from aqueous solution, saline solution, Phosphate-buffered saline (PBS) solution or Dimethyl sulfoxide (DMSO) solution.

The selection of vehicles, excipients and/or diluents suitable for the pharmaceutical composition of the invention can be determined by a person of ordinary skill in the art by using his/her normal knowledge.

The pharmaceutical composition for use according to the invention is suitable for administration via any of the known administration routes, e.g. topical, oral, enteral, parenteral, intrathecal, epidural and intranasal.

As used herein, parenteral administration includes, but is not limited to, administration of a pharmaceutical composition by subcutaneous and intramuscular injection, implantation of sustained release depots, intravenous injection administration.

Intrathecal administration is generally characterized by the direct injection of the drug into the cerebrospinal fluid (CSF) within the intrathecal space of the spinal column.

Injectables can be prepared as liquid solutions or suspensions, solid forms that can be reconstituted into solutions or suspensions prior to injection, or as emulsions. The pharmaceutical composition for use according to the invention may be in the form of a sterile injectable suspension. This suspension may be formulated according to known methods using suitable dispersing or wetting agents and suspending agents. The sterile injectable preparation may also be a sterile injectable solution or suspension in a non-toxic parenterally-acceptable diluent or solvent. Sterile, fixed oils are conventionally employed as a solvent or suspending medium. For this purpose any bland fixed oil may be employed including synthetic mono- or diglycerides, fatty acids (including oleic acid), naturally occurring vegetable oils like sesame oil, coconut oil, peanut oil, cottonseed oil, etc., or synthetic fatty vehicles like ethyl oleate or the like. Buffers, preservatives, antioxidants, and the like can be incorporated as required.

The pharmaceutical composition for use according to the invention may be administered by topical application in a solution, ointment, gel, cream or any local application. The pharmaceutical composition for use according to the invention may also be administered transdermally by means of a drug eluting device, such as gauze, a patch, pad, or a sponge.

Examples of enteral routes include, but is not limited to, oral, mucosal, buccal, rectal and intragastric route.

The pharmaceutical composition for use according to the invention may be in a form suitable for oral use in any acceptable form, such as a tablet, liquid, capsule, powered, syrup, and the like. The pharmaceutical composition intended for oral use may contain one or more agents selected from the group consisting of a sweetening agent such as sucrose, lactose, or saccharin, flavoring agents such as peppermint, oil of wintergreen or cherry, coloring agents and preserving agents in order to provide pharmaceutically elegant and palatable preparations. Tablets contain the compound for use according to the invention in admixture with non-toxic pharmaceutically acceptable excipients. The excipients used may be, for example, (1) inert diluents such as calcium carbonate, lactose, calcium phosphate or sodium phosphate; (2) granulating and disintegrating agents such as corn starch, potato starch or alginic acid; (3) binding agents such as gum tragacanth, corn starch, gelatin or acacia, and (4) lubricating agents such as magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

The pharmaceutical composition according to the invention may also be administered in the form of suppositories for rectal administration of the SERPINA3 inhibitor compound. These compositions may be prepared by mixing the invention compounds with a suitable nonirritating excipient, such as cocoa butter, synthetic glyceride esters of polyethylene glycols, which are solid at ordinary temperatures, but liquefy and/or dissolve in the rectal cavity to release the drug.

For solid dosage forms, non-toxic solid excipients for admixture with compounds for use according to the invention include, but are not limited to, pharmaceutical grades of mannitol, lactose, starch, magnesium stearate, sodium saccharin, polyalkylene glycols, talcum, cellulose, glucose, sucrose, and magnesium carbonate. The solid dosage forms may be coated by a material such as glyceryl monostearate or glyceryl distearate, which is utilized in known techniques to delay disintegration and absorption in the gastrointestinal tract for the purpose of providing a sustained action over a longer period.

Pharmaceutically administrable liquid dosage forms can, for example, comprise a solution or suspension of a compound for use according to the invention and optional pharmaceutical adjutants in a carrier, such as water, saline, aqueous dextrose, glycerol, ethanol and the like. The liquid dosage forms may also contain nontoxic auxiliary substances such as wetting or emulsifying agents, pH buffering agents and the like. Examples of such auxiliary agents include sodium acetate, sorbitan monolaurate, triethanolamine, sodium acetate, triethanolamine oleate, etc. Methods for preparing such dosage forms are well-known to persons skilled in the art.

The pharmaceutical composition for use according to the invention should be administered as frequently as necessary and for as long of a time as necessary in order to achieve the desired effect, i.e. affecting the onset and/or progression of the prion or prion-like disease.

Additionally, the mode of administration of the pharmaceutical composition for use according to the invention may be designed to delay release of the active compound over a given period of time, or to carefully control the amount of drug released at a given time during the course of therapy.

One of ordinary skill in the art can readily determine a suitable course of treatment utilizing the pharmaceutical compositions for use according to the invention.

The following experimental section is provided purely by way of illustration and is not intended to limit the scope of the invention as defined in the appended claims.

### 1. MATERIAL AND METHODS

### 1.1 Maintenance of cell culture

The mouse hypothalamic cell line GT1 and GT1 cells infected with RML prion strain (ScGT1) were cultured in Dulbecco's modified Eagle's medium (DMEM), supplemented with 10% of FBS and 1% penicillin-streptomycin. The mouse neuroblastoma cell line N2a and N2a cells infected with RML prion strain (ScN2a) were cultured in Minimal Essential Medium (MEM) complemented with 10% fetal bovine serum (FBS), 1% non-essential amino acids (NEAA), 1% L-glutamax and 1% penicillin/streptomycin. All cell lines were maintained in a 5% CO₂ atmosphere.

### 1.2 MTT assay for cell viability

Cells were maintained in culture and grown to confluence. The cells were detached and the medium was refreshed. Cell density was adjusted to 2×10⁴ cell/ml with MEM (N2a, ScN2a) or 3×10⁴ with DMEM (GT1, ScGT1) by using Scepter^{™} 2.0 Cell Counter (Millipore). Cells were seeded in a 96-well plate and allowed to settle for 24 hours prior to the addition of the compounds. Each compound - dissolved in DMSO- was diluted in the cell medium to a final concentration of 0.1, 1 and 10 µM. The day after, cell culture medium was refreshed together with the addition of the compound. The plate was incubated for 4 days. The 3-(4,5-dimethylthiazol-2-yl)-2,5- diphenyltetrazolium bromide powder (MTT) (SIGMA) was diluted in phosphate buffer solution (PBS) to a working dilution of 5 mg/mL and added to the medium. Cells were incubated with the MTT solution for 3 hours at 37°C. After incubation, a solution of DMSO/2-Propanol (1:1) was added to each well and the plate was kept at room temperature (RT) for 30 minutes before reading. The emission intensity was quantified using a Spectramax Gemini EM (SoftMax Pro) plate reader, excitation/emission ratio equal to 570/690 nm. MTT assays were performed in triplicate.

### 1.3 In silico screening

Several libraries of compounds have been virtually screened using the Glide program for their potential interaction with the SERPINA3 protein target. A protein site has been identified through superimposition of a SerpinaA3 structural model (obtained by homology modeling) built in house with other two proteins of the same family which have been crystallized with respective inhibitors: human plasminogen activator inhibitor PAI-1 complexed with CDE-096 (PDB ID: 4G8O) and alpha-1-antitrypsin SERPINA1 complexed with citrate (PDB ID: 3CWM) (Figure 13). An initial group of 19 compounds (named A-U) have been selected and tested in vitro. A library of 8 structural analogues (named 1 to 8) of compound G of formula (II) has been identified and tested in vitro (Table I).

**Table I**

| CODE | STRUCTURE | NAME |
|---|---|---|
| Compound 1 | 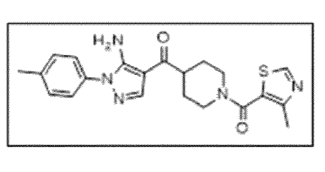 | [4-(5-Amino-1-p-tolyl-1H-pyrazole-4-carbonyl)-piperidin-1-yl]-(4-methyl-thiazol-5-yl)-methanone |
| Compound 2 | 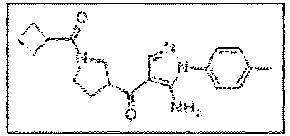 | (5-Aminio-1-p-tolyl-1H-pyrazol-4-yl)-(1-cyclobutanecarbonylpyrrolidin-3-yl)-methanone |
| Compound 3 | 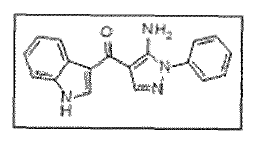 | (5-Amino-1-phenyl-1H-pyrazol-4-yl) -(1H-indol-3-yl)-methanone |
| Compound 4 | 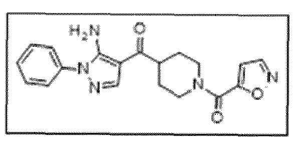 | [4-(5-Amino-1-phenyl-1H-pyrazole-4-carbonyl) -piperidin-1-yl]-isoxazol-5-yl-methanone |
| Compound 5 | 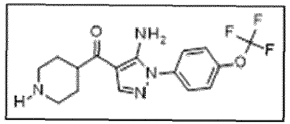 | [5-Amino-1-(4-trifluoromethoxyphenyl)-1H-pyrazol-4-yl]-piperidin-4-ylmethanone |
| Compound 6 | 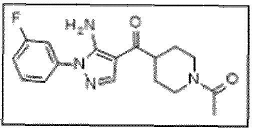 | 1-{4-[5-Amino-1-(3-fluoro-phenyl)-1H-pyrazole-4-carbonyl]-piperidin-1-yl}-ethanone |
| Compound 7 | 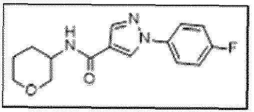 | 1-(4-Fluoro-phenyl)-1H -pyrazole-4-carboxylic acid (tetrahydro-pyran-3-yl)-amide |
| Compound 8 | 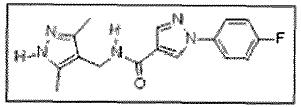 | N-[(3,5-dimethyl-1H-pyrazol-4-yl)methyl]-1-(4-fluorophenyl)-1H-Pyrazole-4-carboxamide |

### 1.4 Drug treatment

All the tested compounds were dissolved in 100% dimethyl sulfoxide (DMSO) to a concentration of 10 mM. This solution was further diluted into a final stock solution of 200µM with PBS. Confluent GT1 and ScGT1 cells were split 1:10 and the day after the drug (20µM) was added to the medium and incubated for 4 days. Confluent N2a and ScN2a cells were split 1:20 and the day after the drug (20µM) was added to the medium and incubated for 4 days. Control cells were treated with the vehicle (≤0.001%DMSO). All the experiments were repeated at least three times.

### 1.5 Western Blot analysis

Cell lysis. After medium removal, cells were rinsed twice with cold PBS and 300 µl of lysis buffer (10 mM Tris-HCl pH 8.0, 150 mM NaCl, 0.5 % nonidet P-40, 0.5 % deoxycholic acid sodium salt) was added directly to the cell plate. Cell lysates were centrifuged at 2000 rpm for 10 minutes at 4°C in a bench microfuge (Eppendorf) and pellet was discarded. Total protein concentration was assessed with BCA assay (bicinchoninic acid method, Euroclone).

PK digestion. 250 µl of 1 mg/ml cell lysates were digested with 25 µg/ml of PK for 1 hour at 37°C. The reaction was stopped with 2 mM of phenylmethylsulphonylfluoride (PMSF) and the PK-digested cell lysates were centrifuged at 55000 rpm for 75 minutes at 4°C in an ultracentrifuge (Beckman Coulter). The pellets were re-suspended in 2X sample loading buffer (125 mM Tris HCl, pH 6.8, 10% 2-mercapethanol, 4% SDS, 0.2% bromophenol blue, 20% glycerol). For the non-PK digested sample, 25 µg of cell lysates were used and 2X loading buffer was added. All the samples were boiled for 10 minutes at 100°C.

SDS page and immunoblotting. Samples were loaded onto 12% Tris-Glycine SDS-PAGE gel and proteins were transferred onto a PVDF membrane (Millipore). The membrane was then blocked with 5% non-fat milk for 1 hour at RT in continuous shaking and then incubated with 1µg/ml monoclonal anti-PrP W226 at 4°C overnight. After 3 washings with TBS-T (Tris 200 mM, NaCl 1.5 mM, 1% Tween-20) the membrane was incubated with goat antimouse IgG F(Ab)₂ conjugated with horseradish peroxidase (HRP) for 1 hour at RT, continuous shaking. Actin beta was detected using monoclonal anti-β-actin-peroxidase antibody (1:10,000 Sigma), SerpinA3n detection was performed using Mouse Serpin A3N Antibody (1:500 R&D Systems) and a rabbit anti-goat HRP secondary antibody. Blots were developed with enhanced chemiluminescent system (ECL, Amersham Biosciences) and visualized on Uvitec Alliance (Cambridge).

### 1.6 De novo prion infection

ScGT1 cells chronically infected with mouse-adapted RML were grown to confluence and 20×10⁶ cells were resuspended in fresh medium and sonicated three times for 15 seconds at 70% amplitude. This solution was incubated with 5×10⁵ GT1 cells seeded on 10 cm petri dish the day before. GT1 cells were grown for 7 days without changing medium. After a week, GT1 cells were split 1:5 with fresh medium for the first passage and for each subsequent passage 1:10. Similarly, chronically RML-infected ScN2a cells were used to infect normal N2a cells. In this case, for the first passage cells were split 1:15 after 4 days and the conditioned medium was added to the cells for additional 4 days. Then, starting from the second passage, cells were split 1:30 with fresh medium. For each passage, the remaining cells were lysed and an aliquot of the cell lysate was subjected to PK-digestion and immunoblot analysis to check the presence of PrP^{Sc}.

### 1.7 Stable transfection with plasmid

Lipofectamine 2000 was used for all plasmid transfection experiments according to the manufacturer's protocol. Briefly, cells were plated in 96-wells plates for each experiment at a density of 1000 cells. Lipofectamine and Optimem medium were mixed and incubated for 5 min, then added to a plasmid and Optimem medium mixture and kept for 15 minutes at room temperature (RT). This solution was added stepwise to cells and gently mixed. Cells were incubated at 37°C overnight, afterwards the medium was replaced by fresh complete medium. Passage cells at a 1:50 into selective medium in the following day. Plasmid for overexpression was pcDNA-SERPINA3N and plasmids for knock-out experiments were pSPCas9n-SERPINA3N-A, pSPCas9n-SERPINA3N-B.

### 2. RESULTS

### 2.1. SerpinA3n expression in GT1, N2a, ScGT1 and ScN2a cell lines

The present inventors investigated the expression of Serpina3n, which is the murine homologue of human SERPINA3, in GT1 and N2a cell lines chronically infected with RML prion strain, designated as ScGT1 and ScN2a, respectively, using non-infected cells as controls. As shown in Figure 1, Serpina3n mRNA levels are strongly increased in ScGT1 cells in comparison with non-infected GT1 cells and in ScN2a cells compared to non-infected N2a. Similarly, increased Serpina3n protein levels were observed in both RML-infected cell lines along with PrP^{Sc} accumulation (Figure 2). These results indicate a strong correlation between Serpina3n and PrP^{Sc} levels.

### 2.2 De novo prion infection of GT1 cell line

To further investigate the correlation between Serpina3n levels and PrP^{Sc} proteins accumulation, the inventors performed de novo prion infection of GT1 cells, monitoring each passage for both protein levels. As shown in Figure 3, the expression of Serpina3n increased together with PrP^{Sc} appearance and accumulation, implying an important role for Serpina3n in prion conversion and/or replication.

### 2.3 De novo prion infection of N2a cell lines overexpressing Serpina3n

As a further assessment of the functional correlation between Serpina3n levels and PrP^{Sc}, the present inventors carried out de novo infection experiments also on N2a cells which stably overexpress Serpina3n. N2a clone 2 cell line - which overexpresses Serpina3n about 7000-fold in comparison to parental N2a cells (Figure 4) - displayed much higher levels (on average 4-fold) of PrP^{Sc} in each single passage if compared to the same passage of parental N2a cell line (Figure 5). These results indicate that overexpression of Serpina3n leads to a faster PrP^{Sc} accumulation and overall to an increased load of prions.

### 2.4 De novo prion infection of Serpina3n knock-out GT1 cell line

*De novo* infection experiments were carried out by the present inventors on GT1 cell line wherein Serpina3n had been stably knock-out. Following infection, Serpina3n knock-out GT1 clone 2 cell line displayed much lower levels (on average 60% less) of PrP^{Sc} in each single passage in comparison to the same passage of parental GT1 cell line (Figure 6). These results indicate that ablation of cellular Serpina3n slows down PrP^{Sc} accumulation, thereby leading to a reduced prion load over time.

### 2.4 Serpina3n knock-out ScN2a cell line

Based on the above-illustrated experimental results, the present inventor generated chronically prion-infected N2a (ScN2a) cell lines knock-out for Serpina3n (Figure 7a and 7b). Notably, all the six cell clones thus obtained showed highly reduced or undetectable PrP^{Sc} levels after few passages in culture (Figure 7c and 7d). These results strongly indicate that Serpina3n expression is crucial for prion replication and that its ablation might be able to cure prions in RML-infected cell lines.

### 2.5 Treatment of ScGT1 cell line with Serpina3n inhibitor compounds

In order to exploit the role of Serpina3n as potential drug target in prion or prion-like diseases, RML ScGT1 cells were treated with the Serpina3 inhibitor compounds which had been selected in silico as above detailed at paragraph 1.3. Among the 19 molecules selected from the first library screening (named A-U), two were discarded because of high citotoxicity. The remaining 17 compounds were tested in in vitro cellular models. Compound G of formula (II) showed the highest efficacy in reducing PrP^{Sc} accumulation of about 50% (Figure 8). These data already show the ability of Serpina3n inhibitor compounds to reduce the PrP^{Sc} amount in cellular models of chronic prion infection. Then, the inventors generated a second library of 8 compounds (named 1-8) which are structural analogs of compound G of formula (II), the most effective among the compounds of the first library. Upon citotoxicity assessment, compound 3 was discarded and the remaining 7 molecules were tested in vitro (Figure 9). Among tested molecules, compound 5 of formula (III) was able to reduce PrP^{Sc} levels of about 60%. Compound 5 of formula (III) was also tested on ScGT1 cells infected with 22L prion strain, obtaining 35% reduction of PrP^{Sc} levels (Figure 10). These results indicate that compound 5 of formula (III) is able to reduce prion load in ScGT1 cell lines infected with different prion strains.

### 2.6 Treatment of ScN2a cell line with Serpina3n inhibitor compounds

Following the results of in vitro test on ScGT1 cells, compound 5 of formula (III) was also administered to RML-infected ScN2a cell line. As shown in Figure 11, upon treatment, a 60% reduction in PrP^{Sc} levels was observed, indicating that the assayed Serpina3 inhibitor compound is able to clear RML prions in different cell lines. Moreover, different drug vehicles were tested in order to assess the solubility of the Serpina3 inhibitor compound in different solvents, namely PBS, H₂0, NaCl and DMSO. As shown in Figure 12, compound 5 of formula (III) was able to reduce PrP^{Sc} in all drug vehicles tested.

### 3. DISCUSSION AND CONCLUSION

The expression of the serine protease inhibitor Serpina3n is strongly increased in prion-infected cells, both at the mRNA and protein level. Notably, the present inventors found that the increase in Serpina3n levels is evident since the pre-clinical stage in mice as well as in de novo prion-infected cell lines, indicating that SERPINA3 plays a central role not only in supporting the progression of prion or prion-like diseases but also in the onset of these disorders. More importantly, the present inventors observed that ablation of the Serpina3n protein - or inhibition of its activity - leads to a significant decrease in the accumulation of PrP^{Sc}.

Without wishing to be bound by any theory, the inventors believe that the SERPINA3 inhibitor compounds for use according to the invention may act by blocking the first step in the serpin-protease interaction that leads to the non-covalent binding of the serpin with the target protease. Indeed, based on the general mechanism which has been described for serpins and proteases, the reaction comprises the following subsequent steps: (i) formation of non-covalent Michaelis-Menten complex between serpin and target protease, (ii) catalytic binding of the protease to the serpin, (iii) formation of the covalent complex, (iv) insertion of the cleaved RCL site in the beta-sheet A, (v) translocation of the protease to the opposite pole of the serpin, (vi) dissociation of the protease.

For other similar complexes, such as the complex formed between human plasminogen activator inhibitor PAI-1 and the inhibitor molecule CDE-096, it has been demonstrated that the inhibitor compound binds the sB/sC pocket between beta sheets B and C, thereby blocking the Michaelis-Menten complex formation between the serpin and the protease. In this way, the RCL region of the serpin cannot longer interact with the active site of the protease, and therefore the serpin protein is sequestered in a conformation in which the RCL region cannot interact with the beta-sheet A. On the other hand, studies carried out on the complex formed between alpha-1-antitrypsin SERPINA1 and citrate have demonstrated that citrate is capable to prevent the aggregation of serpin by blocking the sB/sC pocket and the conformation rearrangements needed for the polymerization.

Finally, the specificity of the SERPINA3 inhibitor compounds for use according to the invention is discussed. Despite the high structural homology among all the serpin proteins, the sB/sC pocket is considered a protein site that can be exploited for modulating the activity of different serpins. Such protein site corresponds to the serpin region which interacts with the respective ligand. Indeed, even if citrate and CDE-096 bind in a similar manner to SERPINA1 and human plasminogen activator inhibitor PAI-1, respectively, they exert different effects - inhibition of aggregation for the citrate, inhibition of protease binding for CDE-096. Moreover, the alignment of the amino acid sequences of human PAI-1, SERPINA1 and SERPINA3 reveals that the sB/sC pocket, i.e, the ligand binding site, is actually one of the least conserved amino acid portions.

So far, no inhibitors against SERPINA3 or the mouse orthologue Serpina3n have been developed for neurodegenerative disorders. Some studies exist describing the use of Serpina3n itself as inhibitor of other enzymes. For instance, Serpina3n has been used to inhibit the activity of granzyme-B in a mouse model of Multiple Sclerosis, showing reduced axonal and neuronal injury.

In conclusion, the present inventors found a new class of small molecules acting as SERPINA3 inhibitors, which are able to reduce PrP^{Sc} accumulation in prion-infected cell lines, thereby representing a new therapeutic treatment against prion and prion-like diseases.

### BIBLIOGRAPHY

1. Barbisin, M. et al. Gene expression profiling of brains from bovine spongiform encephalopathy (BSE)-infected cynomolgus macaques. BMC Genomics 15, 434, doi:10.1186/1471-2164-15-434 (2014).
2. Vanni, S. et al. Differential overexpression of SERPINA3 in human prion diseases. Sci Rep 7, 15637, doi:10.1038/s41598-017-15778-8 (2017).

## Claims

1. A SERPINA3 inhibitor compound, for use in the prevention and/or therapeutic treatment of a prion or prion-like disease in a subject in need thereof, said compound having the general formula (I) 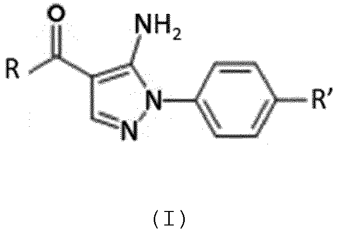 wherein
R is a saturated or unsaturated 5- or 6-membered ring containing at least one heteroatom; and
R' is a halogen atom or a C₁-C₄ alkyloxy group substituted with at least one halogen atom.

2. The SERPINA3 inhibitor compound for use according to claim 1, wherein the heteroatom is nitrogen.

3. The SERPINA3 inhibitor compound for use according to claim 1 or 2, wherein R is a piperidine or pyrrole ring and/or R' is a fluorine atom or a trifluoromethoxy group.

4. The SERPINA3 inhibitor compound for use according to claim 3, having the formula (II):

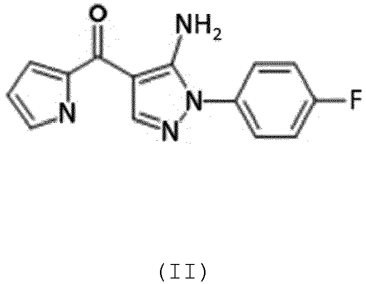

5. The SERPINA3 inhibitor compound for use according to claim 3, having the formula (III):

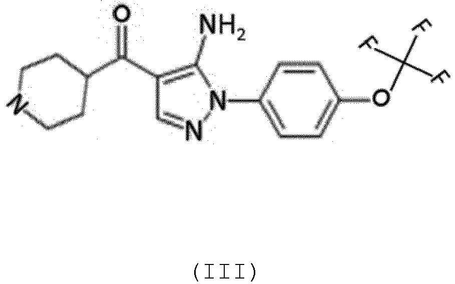

6. The SERPINA3 inhibitor compound for use according to any of claims 1 to 5, wherein the treatment affects the onset and/or progression of said prion or prion-like disease.

7. The SERPINA3 inhibitor compound for use according to any of claims 1 to 6, wherein the subject is a human being.

8. The SERPINA3 inhibitor compound for use according to claim 7, wherein the prion or prion-like disease is selected from the group consisting of Creutzfeldt-Jacob disease (CJD), fatal familial insomnia (FFI), Gerstmann-Straussler-Scheinker syndrome (GSS), Alzheimer's disease, Parkinson's disease, Huntington's Chorea, Amyotrophic lateral sclerosis (ALS) and_Frontotemporal Dementia (FTD).

9. The SERPINA3 inhibitor compound for use according to claim 6, wherein the prion or prion-like disease is selected from the group consisting of ovine scrapie, bovine spongiform encephalopathy (BSE), transmissible mink encephalopathy, feline spongiform encephalopathy, exotic ungulate spongiform encephalopathy, chronic wasting disease of cervids and spongiform encephalopathy of primates.

10. A pharmaceutical composition for use in the prevention and/or therapeutic treatment of a prion or prion-like disease in a subject in need thereof, comprising a therapeutically effective amount of a SERPINA3 inhibitor compound and pharmaceutically acceptable vehicles, excipients and/or diluents, wherein said SERPINA3 inhibitor compound has the general formula (I) 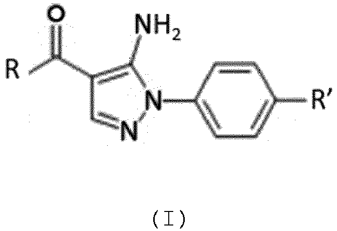 wherein
R is a saturated or unsaturated 5- or 6-membered ring containing at least one heteroatom; and
R' is a halogen atom or a C₁-C₄ alkyloxy group substituted with at least one halogen atom.

11. The pharmaceutical composition for use according to claim 10, wherein the heteroatom is nitrogen.

12. The pharmaceutical composition for use according to claim 10 or 11, wherein R is a piperidine or pyrrole ring and/or R' is a fluorine atom or a trifluoromethoxy group.

13. The pharmaceutical composition for use according to claim 12, wherein the SERPINA3 inhibitor compound has the formula (II)

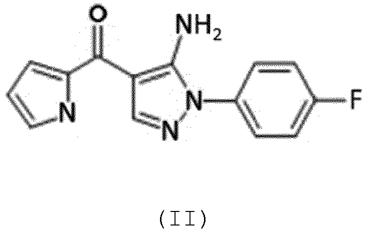

14. The pharmaceutical composition for use according to claim 12, wherein the SERPINA3 inhibitor compound has the formula (III)

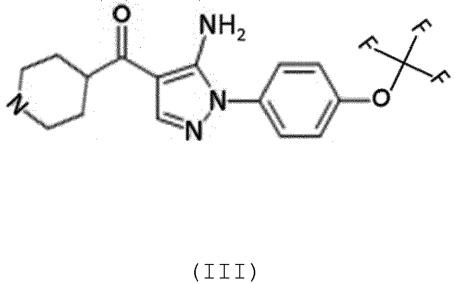

15. The pharmaceutical composition for use according to any of claims 10 to 14, wherein the treatment affects the onset and/or progression of said prion or prion-like disease.

16. The pharmaceutical composition for use according to any of claims 10 to 15, wherein the subject is a human being.

17. The pharmaceutical composition for use according to claim 16, wherein the prion or prion-like disease is selected from the group consisting of Creutzfeldt-Jacob disease (CJD), fatal familial insomnia (FFI), Gerstmann-Straussler-Scheinker syndrome (GSS), Alzheimer's disease, Parkinson's disease, Huntington's Chorea, Amyotrophic lateral sclerosis (ALS) and_Frontotemporal Dementia (FTD).

18. The pharmaceutical composition for use according to claim 15, wherein the prion or prion-like disease is selected from the group consisting of ovine scrapie, bovine spongiform encephalopathy (BSE), transmissible mink encephalopathy, feline spongiform encephalopathy, exotic ungulate spongiform encephalopathy, chronic wasting disease of cervids and spongiform encephalopathy of primates.

19. The pharmaceutical composition for use according to any of claims 10 to 18, which is in a form suitable for topical, oral, enteral, parenteral, intrathecal, epidural or intranasal administration.

20. The pharmaceutical composition for use according to claim 19, which is in a pharmaceutical form suitable for administration by injection.

## Patentansprüche

1. SERPINA3-Inhibitorverbindung zur Verwendung bei der Vorbeugung und/oder therapeutischen Behandlung einer Prionen- oder prionenähnlichen Erkrankung bei einem Subjekt, das diese benötigt, wobei die Verbindung die allgemeine Formel (I) aufweist 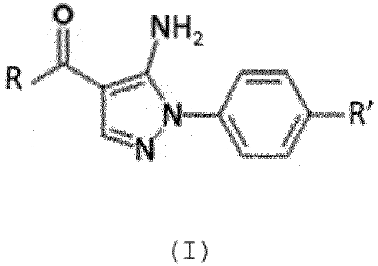 wobei
R ein gesättigter oder ungesättigter 5- oder 6-gliedriger Ring ist, der mindestens ein Heteroatom enthält; und
R' ein Halogenatom oder eine C₁-C₄-Alkyloxygruppe ist, die mit mindestens einem Halogenatom substituiert ist.

2. SERPINA3-Inhibitorverbindung zur Verwendung nach Anspruch 1, wobei das Heteroatom Stickstoff ist.

3. SERPINA3-Inhibitorverbindung zur Verwendung nach Anspruch 1 oder 2, wobei R ein Piperidin- oder Pyrrolring ist und/oder R' ein Fluoratom oder eine Trifluormethoxygruppe ist.

4. SERPINA3-Inhibitorverbindung zur Verwendung nach Anspruch 3, welche die Formel (II) aufweist:

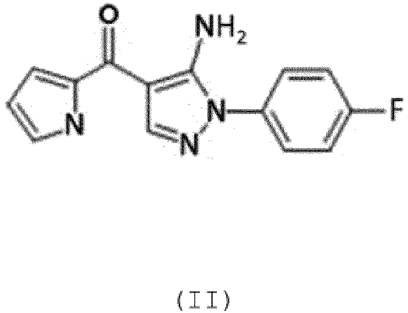

5. SERPINA3-Inhibitorverbindung zur Verwendung nach Anspruch 3, welche die Formel (III) aufweist:

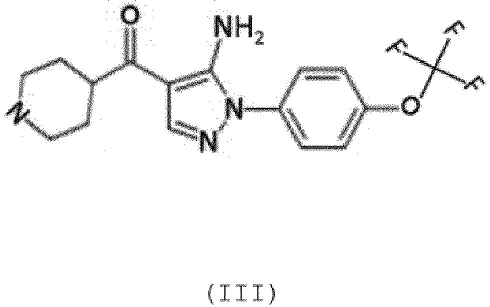

6. SERPINA3-Inhibitorverbindung zur Verwendung nach einem der Ansprüche 1 bis 5, wobei die Behandlung den Ausbruch und/oder das Fortschreiten der Prionen- oder prionenähnlichen Erkrankung beeinflusst.

7. SERPINA3-Inhibitorverbindung zur Verwendung nach einem der Ansprüche 1 bis 6, wobei das Subjekt ein Mensch ist.

8. SERPINA3-Inhibitorverbindung zur Verwendung nach Anspruch 7, wobei die Prionen- oder prionenähnliche Erkrankung aus der Gruppe ausgewählt ist, bestehend aus Creutzfeldt-Jakob-Krankheit (CJD), tödliche familiäre Schlaflosigkeit (FFI), Gerstmann-Sträussler-Scheinker-Syndrom (GSS), Alzheimer-Krankheit, Parkinson-Krankheit, Chorea Huntington, Amyotrophe Lateralsklerose (ALS) und Frontotemporale Demenz (FTD).

9. SERPINA3-Inhibitorverbindung zur Verwendung nach Anspruch 6, wobei die Prionen- oder prionenähnliche Erkrankung aus der Gruppe ausgewählt ist, bestehend aus Scrapie bei Schafen, bovine spongiforme Enzephalopathie (BSE), übertragbare Nerz-Enzephalopathie, spongiforme Enzephalopathie bei Katzen, spongiforme Enzephalopathie bei exotischen Huftieren, chronische Auszehrungskrankheit bei Hirschen und spongiforme Enzephalopathie bei Primaten.

10. Pharmazeutische Zusammensetzung zur Verwendung bei der Vorbeugung und/o- der therapeutischen Behandlung einer Prionen- oder prionenähnlichen Erkrankung bei einem Subjekt, das diese benötigt, umfassend eine therapeutisch wirksame Menge einer SERPINA3-Inhibitorverbindung und pharmazeutisch verträgliche Vehikel, Hilfsstoffe und/oder Verdünnungsmittel, wobei die SERPINA3-Inhibitorverbindung die allgemeine Formel (I) aufweist 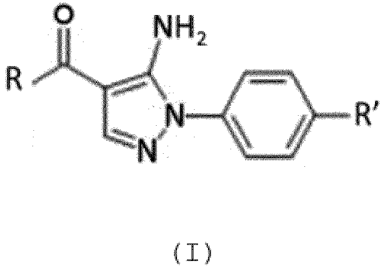 wobei
R ein gesättigter oder ungesättigter 5- oder 6-gliedriger Ring ist, der mindestens ein Heteroatom enthält; und
R' ein Halogenatom oder eine C₁-C₄-Alkyloxygruppe ist, die mit mindestens einem Halogenatom substituiert ist.

11. Pharmazeutische Zusammensetzung zur Verwendung nach Anspruch 10, wobei das Heteroatom Stickstoff ist.

12. Pharmazeutische Zusammensetzung zur Verwendung nach Anspruch 10 oder 11, wobei R ein Piperidin- oder Pyrrolring ist und/oder R' ein Fluoratom oder eine Trifluormethoxygruppe ist.

13. Pharmazeutische Zusammensetzung zur Verwendung nach Anspruch 12, wobei die SERPINA3-Inhibitorverbindung die Formel (II) aufweist

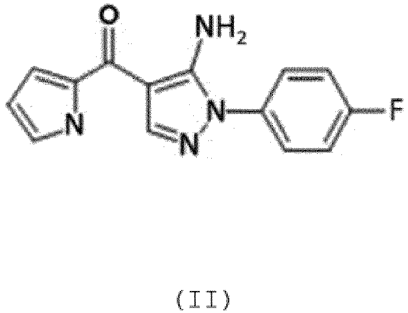

14. Pharmazeutische Zusammensetzung zur Verwendung nach Anspruch 12, wobei die SERPINA3-Inhibitorverbindung die Formel (III) aufweist

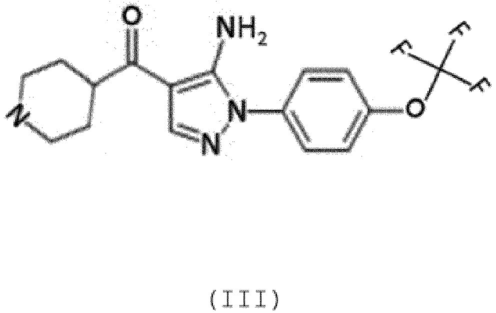

15. Pharmazeutische Zusammensetzung zur Verwendung nach einem der Ansprüche 10 bis 14, wobei die Behandlung den Ausbruch und/oder das Fortschreiten der Prionen- oder prionenähnlichen Erkrankung beeinflusst.

16. Pharmazeutische Zusammensetzung zur Verwendung nach einem der Ansprüche 10 bis 15, wobei das Subjekt ein Mensch ist.

17. Pharmazeutische Zusammensetzung zur Verwendung nach Anspruch 16, wobei die Prionen- oder prionenähnliche Erkrankung aus der Gruppe ausgewählt ist, bestehend aus Creutzfeldt-Jakob-Krankheit (CJD), tödliche familiäre Schlaflosigkeit (FFI), Gerstmann-Sträussler-Scheinker-Syndrom (GSS), Alzheimer-Krankheit, Parkinson-Krankheit, Chorea Huntington, Amyotrophe Lateralsklerose (ALS) und Frontotemporale Demenz (FTD).

18. Pharmazeutische Zusammensetzung zur Verwendung nach Anspruch 15, wobei die Prionen- oder prionenähnliche Erkrankung aus der Gruppe ausgewählt ist, bestehend aus Scrapie bei Schafen, bovine spongiforme Enzephalopathie (BSE), übertragbare Nerz-Enzephalopathie, spongiforme Enzephalopathie bei Katzen, spongiforme Enzephalopathie bei exotischen Huftieren, chronische Auszehrungskrankheit bei Hirschen und spongiforme Enzephalopathie bei Primaten.

19. Pharmazeutische Zusammensetzung zur Verwendung nach einem der Ansprüche 10 bis 18, die in einer Form vorliegt, die zur lokalen, oralen, enteralen, parenteralen, intrathekalen, epiduralen oder intranasalen Verabreichung geeignet ist.

20. Pharmazeutische Zusammensetzung zur Verwendung nach Anspruch 19, die in einer pharmazeutischen Form vorliegt, die zur Verabreichung durch Injektion geeignet ist.

## Revendications

1. Composé inhibiteur de SERPINA3, pour une utilisation dans la prévention et/ou le traitement thérapeutique d'une maladie à prions ou de type prion chez un sujet qui le nécessite, ledit composé ayant la formule générale (I) 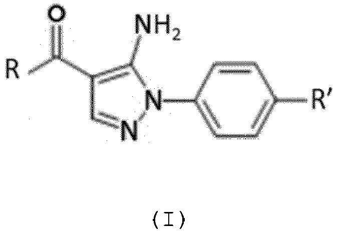 dans laquelle
R est un cycle à 5 ou 6 chaînons, saturé ou insaturé, contenant au moins un hétéroatome ; et
R' est un atome d'halogène ou un groupe alkyloxy en C₁-C₄ substitué par au moins un atome d'halogène.

2. Composé inhibiteur de SERPINA3 pour une utilisation selon la revendication 1, dans lequel l'hétéroatome est de l'azote.

3. Composé inhibiteur de SERPINA3 pour une utilisation selon la revendication 1 ou 2, dans lequel R est un cycle pipéridine ou pyrrole et/ou R' est un atome de fluor ou un groupe trifluorométhoxy.

4. Composé inhibiteur de SERPINA3 pour une utilisation selon la revendication 3, ayant la formule (II) :

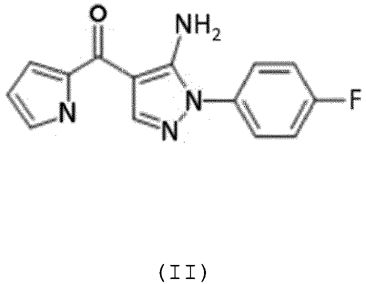

5. Composé inhibiteur de SERPINA3 pour une utilisation selon la revendication 3, ayant la formule (III) :

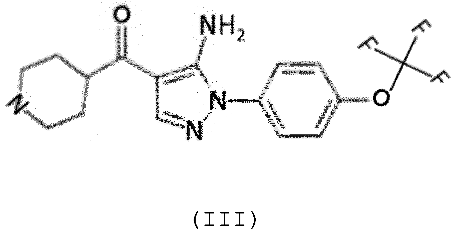

6. Composé inhibiteur de SERPINA3 pour une utilisation selon l'une quelconque des revendications 1 à 5, où le traitement agit sur l'apparition et/ou la progression de ladite maladie à prions ou de type prion.

7. Composé inhibiteur de SERPINA3 pour une utilisation selon l'une quelconque des revendications 1 à 6, où le sujet est un être humain.

8. Composé inhibiteur de SERPINA3 pour une utilisation selon la revendication 7, où la maladie à prions ou de type prion est choisie dans le groupe constitué de la maladie de Creutzfeldt-Jacob (CJD), de l'insomnie fatale familiale (IFF), du syndrome de Gerstmann-Sträussler-Scheinker (GSS), de la maladie d'Alzheimer, de la maladie de Parkinson, de la chorée de Huntington, de la sclérose latérale amyotrophique (SLA) et de la démence frontotemporale (DFT).

9. Composé inhibiteur de SERPINA3 pour une utilisation selon la revendication 6, où la maladie à prions ou de type prion est choisie dans le groupe constitué de la tremblante du mouton, de l'encéphalopathie spongiforme bovine (ESB), de l'encéphalopathie transmissible du vison, de l'encéphalopathie spongiforme féline, de l'encéphalopathie spongiforme des ongulés exotiques, de la maladie débilitante chronique des cervidés et de l'encéphalopathie spongiforme des primates.

10. Composition pharmaceutique pour une utilisation dans la prévention et/ou le traitement thérapeutique d'une maladie à prions ou de type prion chez un sujet qui le nécessite, comprenant une quantité thérapeutiquement efficace d'un composé inhibiteur de SERPINA3 et de véhicules, excipients et/ou diluants pharmaceutiquement acceptables, dans laquelle ledit composé inhibiteur de SERPINA3 a la formule générale (I) 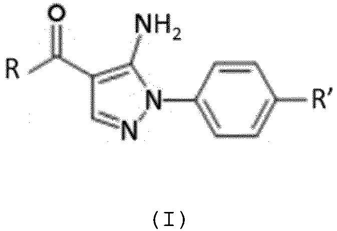 dans laquelle
R est un cycle à 5 ou 6 chaînons, saturé ou insaturé, contenant au moins un hétéroatome ; et
R' est un atome d'halogène ou un groupe alkyloxy en C₁-C₄ substitué par au moins un atome d'halogène.

11. Composition pharmaceutique pour une utilisation selon la revendication 10, dans laquelle l'hétéroatome est l'azote.

12. Composition pharmaceutique pour une utilisation selon la revendication 10 ou 11, dans laquelle R est un cycle pipéridine ou pyrrole et/ou R' est un atome de fluor ou un groupe trifluorométhoxy.

13. Composition pharmaceutique pour une utilisation selon la revendication 12, dans laquelle le composé inhibiteur de SERPINA3 a la formule (II)

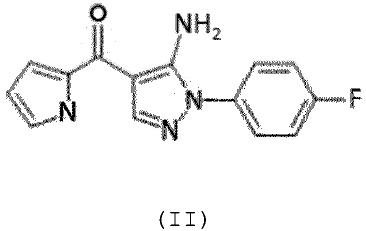

14. Composition pharmaceutique pour une utilisation selon la revendication 12, dans laquelle le composé inhibiteur de SERPINA3 a la formule (III)

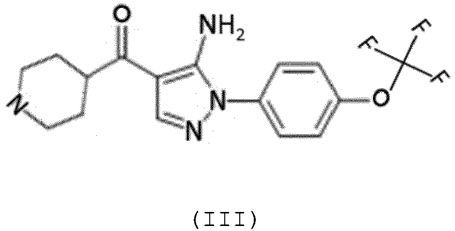

15. Composition pharmaceutique pour une utilisation selon l'une quelconque des revendications 10 à 14, où le traitement agit sur l'apparition et/ou la progression de ladite maladie à prions ou de type prion.

16. Composition pharmaceutique pour une utilisation selon l'une quelconque des revendications 10 à 15, où le sujet est un être humain.

17. Composition pharmaceutique pour une utilisation selon la revendication 16, où la maladie à prions ou de type prion est choisie dans le groupe constitué de la maladie de Creutzfeldt-Jacob (CJD), de l'insomnie fatale familiale (IFF), du syndrome de Gerstmann-Sträussler-Scheinker (GSS), de la maladie d'Alzheimer, de la maladie de Parkinson, de la chorée de Huntington, de la sclérose latérale amyotrophique (SLA) et de la démence frontotemporale (DFT).

18. Composition pharmaceutique pour une utilisation selon la revendication 15, où la maladie à prions ou de type prion est choisie dans le groupe constitué de la tremblante du mouton, de l'encéphalopathie spongiforme bovine (ESB), de l'encéphalopathie transmissible du vison, de l'encéphalopathie spongiforme féline, de l'encéphalopathie spongiforme des ongulés exotiques, de la maladie débilitante chronique des cervidés et de l'encéphalopathie spongiforme des primates.

19. Composition pharmaceutique pour une utilisation selon l'une quelconque des revendications 10 à 18, qui est sous une forme appropriée pour une administration topique, orale, entérale, parentérale, intrathécale, épidurale ou intranasale.

20. Composition pharmaceutique pour une utilisation selon la revendication 19, qui est sous une forme pharmaceutique appropriée pour une administration par injection.
